(19) Europäisches Patentamt
European Patent Office
Office européen des brevets

(11) EP 3 078 701 B1

(12) **EUROPEAN PATENT SPECIFICATION**

(45) Date of publication and mention of the grant of the patent:
**30.01.2019 Bulletin 2019/05**

(21) Application number: **14867983.0**

(22) Date of filing: **05.12.2014**

(51) Int Cl.:
***C08J 3/24*** (2006.01) ***C08L 23/16*** (2006.01)
***C08L 23/26*** (2006.01) ***C08L 61/06*** (2006.01)
***C08L 77/06*** (2006.01)

(86) International application number:
**PCT/JP2014/082224**

(87) International publication number:
**WO 2015/083819 (11.06.2015 Gazette 2015/23)**

(54) **POLYAMIDE THERMOPLASTIC ELASTOMER COMPOSITION AND MOLDED ARTICLE THEREOF**

THERMOPLASTISCHE POLYAMIDELASTOMERZUSAMMENSETZUNG UND FORMARTIKEL DARAUS

COMPOSITION D'ELASTOMERE DE POLYAMIDE THERMOPLASTIQUE ET ARTICLE MOULE CORRESPONDANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.12.2013 JP 2013253036**
**13.02.2014 JP 2014025249**
**28.03.2014 JP 2014068102**

(43) Date of publication of application:
**12.10.2016 Bulletin 2016/41**

(73) Proprietor: **Mitsui Chemicals, Inc.**
**Minato-ku**
**Tokyo 105-7122 (JP)**

(72) Inventors:
- **EBATA, Hiroki**
  **Sodegaura-shi,**
  **Chiba 299-0265 (JP)**
- **ENOMOTO, Tatsuya**
  **Sodegaura-shi,**
  **Chiba 299-0265 (JP)**
- **WASHIO, Isao**
  **Sodegaura-shi,**
  **Chiba 299-0265 (JP)**
- **KAGEYAMA, Fumio**
  **Sodegaura-shi,**
  **Chiba 299-0265 (JP)**
- **TAKEISHI, Atsushi**
  **Ichihara-shi,**
  **Chiba 299-0108 (JP)**
- **ISHII, Yuji**
  **Ichihara-shi,**
  **Chiba 299-0108 (JP)**
- **AMANO, Akinori**
  **Sodegaura-shi,**
  **Chiba 299-0265 (JP)**

(74) Representative: **Hoffmann Eitle**
**Patent- und Rechtsanwälte PartmbB**
**Arabellastraße 30**
**81925 München (DE)**

(56) References cited:
**EP-A1- 1 698 661**
**JP-A- S6 341 554**
**JP-A- 2001 507 060**
**JP-A- 2003 096 245**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 078 701 B1

## Description

### Technical Field

The present invention relates to a polyamide-based thermoplastic elastomer composition excellent in rubber elasticity and moldability, and a molded article thereof.

The present invention also relates to a polyamide-based thermoplastic elastomer composition having good elasticity retention rate at a high temperature, oil resistance and moldability in combination, and a molded article (for example, automobile constant velocity joint boot) obtained from the composition by injection molding or blow molding.

The present invention further relates to a resin composition for use in an industrial tube, which is a polyamide-based thermoplastic elastomer composition excellent in flexibility, impact resistance, fuel and solvent permeation prevention properties, and swelling resistance, as well as an industrial tube having at least a layer including the composition.

### Background Art

A thermoplastic elastomer is a recyclable rubber material, and has been actively investigated in recent years. In addition, while a thermoplastic elastomer exhibits rubber elasticity as in a vulcanized rubber at an ordinary temperature, a matrix phase thereof is plasticized at a high temperature to flow, and therefore a thermoplastic elastomer can also be handled in the same manner as in a thermoplastic resin. Furthermore, a thermoplastic elastomer can more allow for energy saving and an enhancement in productivity than a vulcanized rubber. A thermoplastic elastomer has such characteristics and therefore is increased in demand mainly in various fields of automobiles, building materials, sporting goods, medical equipment components, and industrial components.

Moreover, a thermoplastic elastomer requires no vulcanization step unlike a vulcanized rubber, and has the advantage of being able to be processed in a usual molding machine of a thermoplastic resin. In particular, a polyester-based thermoplastic elastomer is excellent in durability, oil resistance and heat resistance, also has a high elastic modulus to thereby enable to thin a member and well meets the needs for reductions in weight and cost, and therefore is actively studied as an alternative material for an oil resistant rubber.

As a thermoplastic elastomer for use in an application where oil resistance and gas barrier properties are required, a block copolymer is mainly used which includes as a hard segment a crystalline resin, such as polyester and polyamide, synthesized by a polycondensation reaction. For example, there are known a polyester-based elastomer including polyester as a hard segment and polyether as a soft segment, and a polyamide-based elastomer including polyamide as a hard segment and polyether as a soft segment. Such thermoplastic elastomers, however, have the following disadvantages: flexibility is poor, rubber elasticity is not sufficient, and the soft segment cannot be avoided from being intermingled into the hard segment and therefore heat resistance is low. Accordingly, the application to which such thermoplastic elastomers are applicable is limited.

A method of improving flexibility includes a method of increasing the content of the soft segment in the polymer. If the content of the soft segment is high, however, there is the following tendency: oil resistance is deteriorated and heat resistance is further reduced, and furthermore gas barrier properties are remarkably impaired. As other method, there is also known a method of adding a plasticizer made of an organic compound. The polyester-based elastomer and the polyamide-based elastomer, however, are each a crystalline resin and are hardly miscible with (hardly absorb) the plasticizer, and therefore the plasticization effect is small. A bleeding phenomenon of the plasticizer may also be caused in use of a product. For example, when the product is kept in contact with oil for a long period, the plasticizer may be dissolved out in the oil to result in a reduction in flexibility. Furthermore, when the product is placed at a high temperature, the plasticizer may also be volatilized to cause such a failure.

In order to solve such problems, Patent Literature 1 and Patent Literature 2 disclose a thermoplastic elastomer composition in which a core-shell type rubber particle is added to a resin such as a polyester-based elastomer or a polyamide-based elastomer to improve flexibility and compression set properties.

Patent Literature 3 discloses, as a thermoplastic elastomer that has sufficient flexibility while maintaining excellent oil resistance, to improve compression set at a high temperature, a dynamically crosslinked thermoplastic elastomer including a polyamide, and a rubber selected from the group consisting of an acrylic rubber, a nitrile rubber and a polyether rubber.

Patent Literature 4 discloses, as a thermoplastic elastomer having good oil resistance and excellent in chemical resistance and gas barrier properties, a dynamically crosslinked thermoplastic elastomer including an aromatic polyamide and an addition polymerization-based block copolymer.

Meanwhile, a polyester-based thermoplastic elastomer, application development of which is performed in various fields of an automobile component or a mechanical component, is excellent in durability, oil resistance and heat resistance, also has a high elastic modulus to thereby enable to thin a member and well meets the needs for reductions in weight and cost, and therefore is actively studied as an alternative material for an oil resistant rubber.

For example, a chloroprene rubber material has been mainly used as a material for resin flexible boots having a bellows shape because of being relatively inexpensive, having proper flexibility and being excellent in creep properties. In recent years, however, a polyester-based thermoplastic elastomer has progressively replaced such a chloroprene rubber material because of having the advantages of enabling to simplify a production process, being excellent in heat resistance and also having a long durability life as a boot material (Patent Literature 5).

Moreover, a polyamide typified by nylon 6 or nylon 66 is excellent in physical properties such as molding processability, mechanical properties and chemical resistance, and therefore is widely used as a component material in various fields such as automobiles, industrial materials, clothing materials, electrical and electronics, and industry fields.

For example, while a metallic tube has been mainly used as a conventional industrial tube, a resin tube has progressively replaced such a metallic tube in recent years for the purpose of a reduction in weight. In particular, nylon 11 and nylon 12 excellent in flexibility have been frequently used in a tube or hose molded article for automobile fuel piping. A molded article using nylon 11 or nylon 12 is excellent in toughness, chemical resistance and flexibility, but is not sufficient in permeation prevention properties against fuel and alcohols. Accordingly, it has been increasingly difficult for such a molded article to address regulations on fuel gas evaporation with respect to an automobile in recent years. Furthermore, a plasticizer such as butylbenzenesulfonamide (BBSA) to be added in order to make a tube flexible may also be extracted into fuel to cause the tube to be clogged and/or cause the tube itself to be reduced in flexibility.

A multi-layer tube is then proposed in Patent Literature 6, in which an outer layer is configured by an aliphatic polyamide and 9T nylon is stacked as an inner layer. This multi-layer tube is improved in chemical resistance and fuel gas permeation prevention properties.

A multi-layer structure is proposed in Patent Literature 7, in which a composition including an impact resistance modifier (polyolefin-based elastomer) added to 10T10.10 nylon is used for an outer layer and a specific fluorinated polymer is used for an inner layer as a barrier layer.

Polyamide 62 using oxalic acid is proposed as an aliphatic polyamide excellent in gas permeation prevention properties in Patent Literature 8. This polyamide is excellent in fuel permeation prevention properties and tube moldability.

Furthermore, EP 1 698 661 A1 describes a thermoplastic elastomer composition which contains a thermoplastic resin having a polar group and an ethylene·α-olefin elastomer having a functional group. The ethylene·α-olefin elastomer having the functional group is preferably a random copolymer obtained by copolymerizing ethylene, an α-olefin having 3 to 10 carbon atoms, an unsaturated monomer having the functional group and an optional non-conjugated diene.

JP S63-041554 A is concerned with a composition obtained by blending (A) 90-60 pts.wt. of an ethylene·α-olefin copolymer rubber with (B) 10-40 pts.wt. of a polyamide and (C) 0.5-40 pts.wt. of a modified olefin polymer to give 100 pts.wt. of the total amount of the components (A) and (B). Ethylene-propylene copolymer rubber or ethylene-1-butene-polyene copolymer rubber, may be used as the component (A). A modified olefin polymer having functional groups or a phenolic compound linked to a novolak prepolymer may be used as the component (C).

## Citation List

### Patent Literatures

Patent Literature 1: JP8-231770A
Patent Literature 2: JP2011-148887A
Patent Literature 3: WO2006/003973
Patent Literature 4: JP2004-217698A
Patent Literature 5: JP2001-173672A
Patent Literature 6: WO2005/102694
Patent Literature 7: JP2012-224085A
Patent Literature 8: JP2013-95802A


## Summary of Invention

### Technical Problem

The thermoplastic elastomers described in Patent Literatures 1 to 4, however, have been found to be insufficient in respective specific characteristics.

For example, the composition described in Patent Literature 1 and Patent Literature 2 is not sufficiently improved in flexibility, and also has the problem about moldability (fluidity) thereof. Furthermore, the rubber component in the composition is difficult to sufficiently disperse, and therefore when the composition is subjected to extrusion molding or

blow molding, the surface appearance of the resulting molded article tends to be poor.

The composition described in Patent Literature 3 is poor in moldability, and is not sufficient also in compression set resistance. In addition, the composition requires specialized rubber material and crosslinking agent, and therefore is low in general versatility.

The composition described in Patent Literature 4 has difficulty in controlling the reaction in the step of dynamic crosslinking because the aromatic polyamide used therein has a high melting point of 317°C. In addition, the molding temperature of the composition is high and therefore it is difficult to perform extrusion molding or blow molding. Furthermore, as seen from Examples and Comparative Examples in Patent Literature 4, crosslinking causes an increase in hardness (rubber hardness) to make the control of flexibility difficult, and sufficient flexibility is hardly achieved by dynamic crosslinking.

A polyester-based thermoplastic elastomer is, however, reduced in elastic modulus with being made flexible, when used at a high temperature, and therefore has the problem of being low in elasticity retention rate at a high temperature. In addition, the elastomer also has the problem of being remarkably reduced in mechanical strength of a product at a high temperature in particular by hydrolysis.

On the other hand, a material for boots has been demanded to have higher heat resistance. The reason for this is considered to be, for example, the change in design of a configuration of a vehicle (for example, installation of a vehicle undercover) in accordance with a specification change of an engine (introductions of a supercharging system and an exhaust recirculation system) and an enhancement in fuel efficiency. A polyester-based thermoplastic elastomer, which is low in elasticity retention rate at a high temperature and poor in hydrolysis resistance, increasingly has difficulty in satisfying the requirement for higher heat resistance.

In the multi-layer tube in Patent Literature 6, 9T nylon is extremely hard (high in elastic modulus) like a conventional material, and therefore a plasticizer and an elastomer are required to be added. As a result, the problems about extraction of the plasticizer and fuel gas permeation prevention properties are not solved.

In the multi-layer structure in Patent Literature 7, the outer layer has sufficient pliability (flexibility) and zinc chloride resistance in combination. However, the performances of the resin of barrier layer (fluorinated polymer layer) as the inner layer are insufficient, and the barrier layer is required to have a multi-layer structure as in a conventional material. As a result, flexibility of the multi-layer structure is not sufficient.

The aliphatic polyamide in Patent Literature 8 is extremely hard (high in elastic modulus), and therefore a large amount of a plasticizer is required. As a result, there is the problem about extraction of the plasticizer.

The present invention has been made in order to solve the problems of the related arts. That is, an object of the present invention is to provide a polyamide-based thermoplastic elastomer composition excellent in rubber elasticity such as flexibility, compression set resistance and elongation and also excellent in moldability such as extrusion moldability, and a molded article thereof.

Another object of the present invention is to provide a polyamide-based thermoplastic elastomer composition having good elasticity retention rate at a high temperature, oil resistance and moldability in combination, and a molded article thereof.

Further object of the present invention is to provide a resin composition having various performances required for an industrial tube, namely, sufficient flexibility and impact resistance even in no use of a large amount of a plasticizer, and also being excellent in fuel and solvent permeation prevention properties, and swelling resistance, as well as an industrial tube using the resin composition.

**Solution to Problem**

The present invention includes the following aspects [1] to [19].

[1] A polyamide-based thermoplastic elastomer composition [Y], in which a rubber composition [X] and a phenol resin-based crosslinking agent [IV] are dynamically crosslinked,
wherein the rubber composition [X] comprises:

a polyamide [I] comprising 30 to 100 % by mole of a structural unit derived from terephthalic acid
in total structural units of dicarboxylic acids and a melting point (Tm) determined by differential scanning calorimetry (DSC) of the polyamide being 220 to 290°C,
an ethylene-α-olefin-unconjugated polyene copolymer rubber [II] comprising structural units derived from ethylene [a], an α-olefin [b] having 3 to 20 carbon atoms, and an unconjugated polyene [c] having at least one carbon-carbon double bond in one molecule, respectively, and
an olefin-based polymer [III] comprising 0.3 to 5.0% by mass of a functional group structural unit in a molecule.

[2] The polyamide-based thermoplastic elastomer composition [Y] according to [1], wherein the polyamide [I] further

comprises a structural unit derived from isophthalic acid as a component of the dicarboxylic acid, and a structural unit derived from an aliphatic diamine having 4 to 15 carbon atoms as a diamine component.

[3] The polyamide-based thermoplastic elastomer composition [Y] according to [2], wherein a molar ratio of the structural unit derived from terephthalic acid/the structural unit derived from isophthalic acid in the polyamide [I] is 65/35 to 50/50.

[4] The polyamide-based thermoplastic elastomer composition [Y] according to any of [1] to [3], wherein 40 to 100% by mole of a total of a diamine component comprised in the polyamide [I] is a structural unit derived from 1,6-hexanediamine.

[5] The polyamide-based thermoplastic elastomer composition [Y] according to any of [1] to [4], wherein the functional group structural unit in the olefin-based polymer [III] comprises a functional group selected from the group consisting of a carboxylic acid group, an ester group, an ether group, an aldehyde group and a ketone group.

[6] The polyamide-based thermoplastic elastomer composition [Y] according to any of [1] to [4], wherein the functional group structural unit in the olefin-based polymer [III] is a maleic anhydride structural unit.

[7] The polyamide-based thermoplastic elastomer composition [Y] according to any of [1] to [6], comprising the polyamide [I] as a matrix component, and a dispersion component dispersed in the matrix component, the dispersion component being a particle including the ethylene-$\alpha$-olefin-unconjugated polyene copolymer rubber [II] and the olefin-based polymer [III], and the dispersion component being satisfied the following (1):

(1) a cross sectional area of the particle, in which a particle size is 5 $\mu$m or more, of the dispersion component is measured, and a proportion of a cumulative cross sectional area of the area of the particle to an entire cross sectional area analyzed is 10% or less.

[8] The polyamide-based thermoplastic elastomer composition [Y1] according to any of [1] to [6], in which a rubber composition [X] comprising:

10 to 60% by mass of the polyamide [I];
30 to 86% by mass of the ethylene-$\alpha$-olefin-unconjugated polyene copolymer rubber [II]; and
3 to 30% by mass of the olefin-based polymer [III], and

1 to 10% by mass of the phenol resin-based crosslinking agent [IV] are dynamically crosslinked, provided that a total amount of [I], [II], [III] and [IV] is 100% by mass.

[9] The polyamide-based thermoplastic elastomer composition [Y1] according to [8], wherein a mass ratio ([II]/[III]) of the ethylene-$\alpha$-olefin-unconjugated polyene copolymer rubber [II] to the olefin-based polymer [III] in the rubber composition [X] is 95/5 to 60/40.

[10] The polyamide-based thermoplastic elastomer composition [Y1] according to [8] and [9], wherein 10% or more of an end group of a molecular chain of the polyamide [I] is terminated by an end-terminating agent, and an amount of a terminal amino group of the molecular chain is 0.1 to 100 mmol/kg.

[11] A molded article obtained from the polyamide-based thermoplastic elastomer composition [Y1] according to any of [8] to [10].

[12] The polyamide-based thermoplastic elastomer composition [Y2] according to any of [1] to [6], in which a rubber composition [X] comprising:

30 to 87.7% by mass of the polyamide [I];
10 to 45% by mass of the ethylene-$\alpha$-olefin-unconjugated polyene copolymer rubber [II]; and
2 to 20% by mass of the olefin-based polymer [III], and

0.3 to 5.0% by mass of the phenol resin-based crosslinking agent [IV] are dynamically crosslinked, provided that a total amount of [I], [II], [III] and [IV] is 100% by mass.

[13] An industrial tube having at least a layer comprising the polyamide-based thermoplastic elastomer composition [Y2] according to [12].

[14] The industrial tube according to [13], which is a tube for automobile piping, a pneumatic tube, a hydraulic tube, a paint spray tube or a medical tube.

[15] The industrial tube according to [13] and [14], having an outer diameter of 2 mm to 50 mm and a thickness of 0.2 mm to 10 mm.

[16] The industrial tube according to any of [13] to [15], further comprising a layer comprising at least one resin selected from the group consisting of a fluororesin, a high density polyethylene resin, a polybutylene naphthalate resin (PBN), an aliphatic polyamide resin, an aromatic polyamide resin, a meta-xylene group-containing polyamide resin, an ethylene-vinyl acetate copolymer saponified product (EVOH) and a polyphenylene sulfide resin (PPS).

[17] A polyamide-based thermoplastic elastomer composition [Z] comprising 10 to 35% by mass of a polyamide [Z1] having a melting point (Tm) determined by differential scanning calorimetry (DSC) of 210 to 270°C and a molten heat capacity ($\Delta$H) determined by DSC of 45 to 80 mJ/mg, and 65 to 90% by mass of a polyamide-based resin composition [Z2], provided that a total amount of [Z1] and [Z2] is 100% by mass, wherein
the polyamide-based resin composition [Z2] is the polyamide-based thermoplastic elastomer composition [Y2] according to [12].
[18] A molded article obtained from the polyamide-based thermoplastic elastomer composition [Z] according to [17] by injection molding or blow molding.
[19] An automobile constant velocity joint boot comprising the polyamide-based thermoplastic elastomer composition [Z] according to [17].

**Advantageous Effect of Invention**

The present invention can provide polyamide-based thermoplastic elastomer compositions [Y], [Yl], [Y2] and [Z] excellent in rubber elasticity such as flexibility, compression set resistance and elongation and also excellent in moldability such as extrusion moldability, and molded articles thereof.

**Brief Description of Drawing**

Figure 1 shows an image example obtained by subjecting a transmission micrograph image of a test piece extrusion-molded, to a binarization process.

**Description of Embodiments**

<Polyamide-based thermoplastic elastomer composition [Y]>

The polyamide-based thermoplastic elastomer composition [Y] for use in the present invention is a composition in which a rubber composition [X] containing components [I], [II] and [III], and a crosslinking agent [IV], described below, are dynamically crosslinked.

<Polyamide [I]>

The polyamide [I] for use in the present invention is a polyamide including 30 to 100% by mole of a structural unit derived from terephthalic acid in total structural units of dicarboxylic acids and a melting point (Tm) of the polyamide determined by differential scanning calorimetry (DSC) being 220 to 290°C.
The structural unit derived from terephthalic acid is a unit represented by the following formula [I-A].

Formula 1

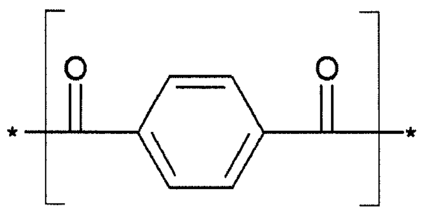

[I-A]

The polyamide [I] is obtained by a condensation polymerization reaction of a dicarboxylic acid component and a diamine component, and includes a dicarboxylic acid structural unit and a diamine structural unit in the molecule. The proportion of the structural unit derived from terephthalic acid in the total 100% by mole of dicarboxylic acid structural units in the molecule of the polyamide [I] is 30 to 100% by mole, preferably 40 to 100% by mole, more preferably 50 to 100% by mole. The polyamide [I] includes the structural unit derived from terephthalic acid to thereby have an increased crystallinity, resulting in an enhancement in physical properties such as heat resistance. Herein, the polyamide [I] is preferably a semi-aromatic polyamide partially containing an aliphatic skelton.
As the dicarboxylic acid component constituting the polyamide [I], an aromatic dicarboxylic acid other than terephthalic acid, and an aliphatic dicarboxylic acid can be used together with terephthalic acid in combination.
Examples of the aromatic dicarboxylic acid other than terephthalic acid include isophthalic acid, 2,6-naphthalenedicarboxylic acid, 2,7-naphthalenedicarboxylic acid, 1,4-naphthalenedicarboxylic acid, 1,4-phenylenedioxydiacetic acid, 1,3-phenylenedioxydiacetic acid, diphenic acid, diphenylmethane-4,4'-dicarboxylic acid, diphenylsulfone-4,4'-dicarboxylic acid and 4,4'-biphenyldicarboxylic acid. These can also be used in combinations of two or more. Among them, isophthalic acid is preferable from the viewpoint of an increase in melt tension. The content of a structural unit derived from the aromatic dicarboxylic acid (such as isophthalic acid) other than terephthalic acid in a total of the dicarboxylic acid structural units is preferably 0 to 50% by mole, more preferably 0 to 40% by mole.

The aliphatic dicarboxylic acid is preferably an aliphatic dicarboxylic acid having 4 to 12 carbon atoms, such as succinic acid, glutaric acid, adipic acid, pimelic acid, suberic acid, azelaic acid and sebacic acid. These can also be used in combinations of two or more. Among them, an adipic acid is particularly preferable in terms of cost and dynamic properties. The content of a structural unit derived from the aliphatic dicarboxylic acid in a total of the dicarboxylic acid structural units is preferably 0 to 50% by mole, more preferably 0 to 40% by mole.

The diamine component constituting the polyamide [I] is preferably an aliphatic diamine. The number of carbon atoms in the aliphatic diamine is preferably 4 to 12, more preferably 6 to 9. Specific examples of the aliphatic diamine include straight-chain aliphatic diamines such as 1,4-diaminobutane, 1,6-diaminohexane, 1,7-diaminoheptane, 1,8-diaminooctane, 1,9-diaminononane, 1,10-diaminodecane, 1,11-diaminoundecane and 1,12-diaminododecane; and chain aliphatic diamines having a side chain, such as 2-methyl-1,5-diaminopentane, 2-methyl-1,6-diaminohexane, 2-methyl-1,7-diaminoheptane, 2-methyl-1,8-diaminooctane, 2-methyl-1,9-diaminononane, 2-methyl-1,10-diaminodecane and 2-methyl-1,11-diaminoundecane. These can also be used in combinations of two or more. Among them, 1,6-diaminohexane (= hexamethylenediamine) is preferable.

As a component constituting the polyamide [I], an aminocarboxylic acid having amine moiety and carboxylic acid moiety in the molecule in combination may also be used. Specific examples of the aminocarboxylic acid include straight-chain aminocarboxylic acids such as 4-aminobutyric acid, 5-aminoheptanoic acid, 6-aminohexanoic acid, 7-aminoheptanoic acid, 11-aminoundecanoic acid and 12-aminododecanoic acid. These can also be used in combinations of two or more. Among them, 11 -aminoundecanoic acid and 12-aminododecanoic acid are preferable.

The polyamide [I] can be produced according to a conventionally known method. For example, the dicarboxylic acid component including terephthalic acid can be subjected to a condensation polymerization reaction with the diamine component in a solution.

The polyamide [I] is preferably terminated at least a part of end groups of the morecular chain with an end-terminating agent. In particular, the proportion of the end group terminated by the end-terminating agent is preferably 10% or more, more preferably 40% or more, particularly preferably 60% or more, most preferably 75% or more in terms of melting stability, heat resistance and hydrolysis resistance. In addition, the amount of the terminal amino group in the molecular chain is preferably 0.1 to 100 mmol/kg, more preferably 0.1 to 30 mmol/kg.

The end- terminating agent is not particularly limited as long as it is a monofunctional compound having reactivity with an amino group or a carboxyl group at the polyamide ends, and is preferably a monocarboxylic acid or a monoamine in terms of reactivity and stability of the end terminated, and is more preferably a monocarboxylic acid in terms of ease of handling. Additionally, acid anhydride monoisocyanates, monoacid halides, monoesters, and monoalcohols can also be used.

The monocarboxylic acid for use as the end-terminating agent is not particularly limited as long as it has reactivity with an amino group. Specific examples thereof include aliphatic monocarboxylic acids such as acetic acid, propionic acid, butyric acid, valeric acid, caproic acid, caprylic acid, lauric acid, tridecylic acid, myristic acid, palmitic acid, stearic acid, pivalic acid and isobutyric acid; alicyclic monocarboxylic acids such as cyclohexanecarboxylic acid; and aromatic monocarboxylic acids such as benzoic acid, toluic acid, $\alpha$-naphthalenecarboxylic acid, $\beta$-naphthalenecarboxylic acid, methylnaphthalenecarboxylic acids and phenylacetic acid. These can also be used in combinations of two or more. Among them, acetic acid, propionic acid, butyric acid, valeric acid, caproic acid, caprylic acid, lauric acid, tridecylic acid, myristic acid, palmitic acid, stearic acid and benzoic acid are further preferable in terms of reactivity, stability of the end terminated, and price.

The monoamine for use as the end-terminating agent is not particularly limited as long as it has reactivity with a carboxyl group. Specific examples thereof include aliphatic monoamines such as methylamine, ethylamine, propylamine, butylamine, hexylamine, octylamine, decylamine, stearylamine, dimethylamine, diethylamine, dipropylamine and dibutylamine; alicyclic monoamines such as cyclohexylamine and dicyclohexylamine; and aromatic monoamines such as aniline, toluidines, diphenylamines and naphthylamines. These can also be used in combinations of two or more. Among them, butylamine, hexylamine, octylamine, decylamine, stearylamine, cyclohexylamine and aniline are more preferable in terms of reactivity, boiling point, stability of the end terminated, and price.

The amount of the end-terminating agent for use in production of the polyamide [I], to be used, can be determined from the relative viscosity of the polyamide finally obtained, and the termination rate of the end group. A specific amount to be used is changed depending on the reactivity, boiling point, reaction apparatus, and reaction conditions of the end-terminating agent used, and is usually in the range from 0.3 to 10% by mol based on the total molar number of the dicarboxylic acid and the diamine as raw materials.

The melting point (Tm) determined by differential scanning calorimetry (DSC) of the polyamide [I] is 220 to 290°C, preferably 230 to 280°C, more preferably 240 to 280°C. The polyamide [I] has such a melting point (Tm) to thereby exhibit excellent moldability. This melting point (Tm) is measured under the following conditions. First, the polyamide [I] is heated and held once at 320°C for 5 minutes, and then cooled to 23°C at a rate of 10°C/min and thereafter heated at a rate of 10°C/min. The endothermic peak based on fusion here corresponds to the melting point (Tm) of the polyamide [I].

The molten heat capacity (ΔH) of the polyamide [I], determined by DSC, is 10 to 44 mJ/mg, preferably 15 to 40 mJ/mg, more preferably 20 to 40 mJ/mg.

The melt flow rate (T + 10°C, 2.16 kg) of the polyamide [I] is preferably 1 to 300 g/10 min, more preferably 5 to 250 g/10 min. This melt flow rate is a value obtained by measurement according to ASTM D1238 procedure B. Here, T stands for the fusion end temperature (T) determined by differential scanning calorimetry (DSC). The fusion end temperature (T) refers to a temperature at which the absorption of heat based on fusion is completed in the same DSC as in measurement of the melting point. Specifically, the fusion end temperature (T) refers to a temperature at which the endothermic peak observed in DSC is returned to the baseline.

The intrinsic viscosity [η] of the polyamide [I] is preferably 0.5 to 1.6 dl/g, more preferably 0.6 to 1.4 dl/g, particularly preferably 0.6 to 1.4 dl/g. When the intrinsic viscosity [η] is in the above range, the fluidity in molding of the resin composition can be increased, and mechanical properties of the resulting molded product are also improved. This intrinsic viscosity [η] is a value obtained by measurement in 96.5% sulfuric acid at a temperature of 25°C.

<Ethylene-α-olefin-unconjugated polyene copolymer rubber [II]>

The ethylene-α-olefin-unconjugated polyene copolymer rubber [II] for use in the present invention is a copolymer rubber including structural units derived from ethylene [a], an α-olefin [b] having 3 to 20 carbon atoms, and an unconjugated polyene [c] having at least one carbon-carbon double bond in one molecule, which is polymerizable by a metallocene-based catalyst, respectively.

(α-Olefin [b] having 3 to 20 carbon atoms)

Specific examples of the α-olefin [b] having 3 to 20 carbon atoms, constituting the copolymer rubber [II], include propylene, 1-butene, 1-pentene, 1-hexene, 4-methyl-1-pentene, 1-heptene, 1-octene, 1-decene, 1-dodecene, 1-tetradecene, 1-hexadecene and 1-eicosene. Among them, α-olefins having 3 to 8 carbon atoms such as propylene, 1-butene, 1-hexene and 1-octene are preferable. The copolymer rubber [II] can also include structural units derived from two or more of the α-olefins [b]. These α-olefins [b] above are preferable because of not only being relatively inexpensive in raw material costs and excellent in copolymerization properties, but also imparting excellent mechanical properties and good flexibility to the copolymer rubber [II].

(Unconjugated polyene [c])

As the unconjugated polyene [c] having at least one carbon-carbon double bond in one molecule constituting the copolymer rubber [II], which is polymerizable by a metallocene-based catalyst, for example, an aliphatic polyene or an alicyclic polyene can be used.

Specific examples of the aliphatic polyene include 1,4-hexadiene, 1,5-heptadiene, 1,6-octadiene, 1,7-nonadiene, 1,8-decadiene, 1,12-tetradecadiene, 3-methyl-1,4-hexadiene, 4-methyl-1,4-hexadiene, 5-methyl-1,4-hexadiene, 4-ethyl-1,4-hexadiene, 3,3-dimethyl-1,4-hexadiene, 5-methyl-1,4-heptadiene, 5-ethyl-1,4-heptadiene, 5-methyl-1,5-heptadiene, 6-methyl-1,5-heptadiene, 5-ethyl-1,5-heptadiene, 4-methyl-1,4-octadiene, 5-methyl-1,4-octadiene, 4-ethyl-1,4-octadiene, 5-ethyl-1,4-octadiene, 5-methyl-1,5-octadiene, 6-methyl-1,5-octadiene, 5-ethyl-1,5-octadiene, 6-ethyl-1,5-octadiene, 6-methyl-1,6-octadiene, 7-methyl-1,6-octadiene, 6-ethyl-1,6-octadiene, 6-propyl-1,6-octadiene, 6-butyl-1,6-octadiene, 4-methyl-1,4-nonadiene, 5-methyl-1,4-nonadiene, 4-ethyl-1,4-nonadiene, 5-ethyl-1,4-nonadiene, 5-methyl-1,5-nonadiene, 6-methyl-1,5-nonadiene, 5-ethyl-1,5-nonadiene, 6-ethyl-1,5-nonadiene, 6-methyl-1,6-nonadiene, 7-methyl-1,6-nonadiene, 6-ethyl-1,6-nonadiene, 7-ethyl-1,6-nonadiene, 7-methyl-1,7-nonadiene, 8-methyl-1,7-nonadiene, 7-ethyl-1,7-nonadiene, 5-methyl-1,4-decadiene, 5-ethyl-1,4-decadiene, 5-methyl-1,5-decadiene, 6-methyl-1,5-decadiene, 5-ethyl-1,5-decadiene, 6-ethyl-1,5-decadiene, 6-methyl-1,6-decadiene, 6-ethyl-1,6-decadiene, 7-methyl-1,6-decadiene, 7-ethyl-1,6-decadiene, 7-methyl-1,7-decadiene, 8-methyl-1,7-decadiene, 7-ethyl-1,7-decadiene, 8-ethyl-1,7-decadiene, 8-methyl-1,8-decadiene, 9-methyl-1,8-decadiene, 8-ethyl-1,8-decadiene, 6-methyl-1,6-undecadiene and 9-methyl-1,8-undecadiene, and also α,ω-dienes such as 1,7-octadiene and 1,9-decadiene. These can also be used in combinations of two or more. Among them, 7-methyl-1,6-octadiene is preferable.

Specific examples of the alicyclic polyene include 5-ethylidene-2-norbornene (ENB), 5-propylidene-2-norbornene, 5-butylidene-2-norbornene and also 5-vinyl-2-norbornene (VNB); 5-alkenyl-2-norbornenes such as 5-allyl-2-norbornene; and 2,5-norbornadiene, dicyclopentadiene (DCPD), norbornadiene and tetracyclo[4,4,0,$1^{2.5}$,$1^{7.10}$]deca-3,8-diene. Among them, 5-ethylidene-2-norbornene (ENB) is preferable. Examples of other alicyclic polyenes also include 2-methyl-2,5-norbornadiene and 2-ethyl-2,5-norbornadiene. The copolymer rubber [II] may also include a structural unit derived from two or more of the unconjugated polyenes [c].

The copolymer rubber [II] can be synthesized by, for example, the method described in JP2010-241897A.

The proportion of the structural unit derived from ethylene [a] in 100% by mass of the total structural units of the copolymer rubber [II] is preferably 50 to 89% by mass, more preferably 55 to 83% by mass. The proportion of the structural unit derived from the α-olefin [b] having 3 to 20 carbon atoms is preferably 10 to 49% by mass, more preferably 15 to 43% by mass. The proportion of the structural unit derived from the unconjugated polyene [c] is preferably 1 to 20% by mass, more preferably 2 to 15% by mass.

The intrinsic viscosity [η] of the copolymer rubber [II] is preferably 0.5 to 5.0 dl/g, more preferably 1.0 to 4.5 dl/g, particularly preferably 1.5 to 4.0 dl/g. This intrinsic viscosity [η] is a value obtained by measurement in decalin at a temperature of 135°C, and can be determined by measurement according to ASTM D 1601.

<Olefin-based polymer [III]>

The olefin-based polymer [III] for use in the present invention is an olefin-based polymer including 0.3 to 5.0% by mass of a functional group structural unit. Examples of this olefin-based polymer [III] include a modified polyolefin ([III]-1) obtained by reacting a compound having a functional group to thereby introduce the functional group into the polyolefin molecular chain, and a functional group-containing olefin-based copolymer ([III]-2) obtained by copolymerizing an olefin monomer and a monomer having a functional group.

Specific examples of the polyolefin constituting the modified polyolefin ([III]-1) include low density polyethylenes, medium density polyethylenes, high density polyethylenes, polypropylenes, ethylene-propylene copolymers and ethylene-butene copolymers.

As the compound having a functional group, constituting the modified polyolefin ([III]-1), for example, an unsaturated carboxylic acid or a derivative thereof can be used. Specific examples of the unsaturated carboxylic acid or derivative thereof include unsaturated carboxylic acids such as acrylic acid, methacrylic acid, α-ethylacrylic acid, maleic acid, fumaric acid, itaconic acid, citraconic acid, tetrahydrophthalic acid, methyltetrahydrophthalic acid and endocis-bicyclo[2,2,1]hept-5-ene-2,3-dicarboxylic acid (Nadic acid [trademark]), and derivatives thereof such as acid halides, amides, imides, acid anhydrides and esters thereof. Among them, an unsaturated dicarboxylic acid or an acid anhydride thereof is preferable, maleic acid, Nadic acid (trademark) or an acid anhydride thereof is more preferable, and maleic anhydride is particularly preferable. Maleic anhydride is relatively high in reactivity with a polyolefin before modification, hardly causes polymerization of maleic anhydride, and tends to be stable as a basic structure. Therefore, maleic anhydride has various advantages such as production of a stable-quality modified polyolefin ([III]-1).

A preferable mode of the modified polyolefin ([III]-1) is a modified ethylene-α-olefin copolymer. The density of this modified ethylene-α-olefin copolymer is preferably 0.80 to 0.95 g/$cm^3$, more preferably 0.85 to 0.90 g/$cm^3$.

Examples of the functional group-containing olefin-based copolymer ([III]-2) include a copolymer of ethylene and a monomer having a functional group, such as an acryl-based monomer or a vinyl monomer. Specific examples include an ethylene-vinyl acetate-maleic anhydride copolymer (e.g., Orevac (registered trademark) produced by Arkema) and an ethylene-acrylic acid ester-functional acrylic acid ester (e.g., glycidyl acrylate or glycidyl methacrylate) copolymer (e.g., Lotader (registered trademark) produced by Arkema).

The intrinsic viscosity [η] of the olefin-based polymer [III], measured in a decalin (decahydronaphthalene) solution at 135°C, is preferably 0.5 to 4.0 dl/g, more preferably 0.7 to 3.0 dl/g, particularly preferably 0.8 to 2.5 dl/g. When the [η] is in the above range, toughness and melt fluidity of the resin composition can be simultaneously satisfied at high levels. This intrinsic viscosity [η] is measured as follow according to an ordinary method. Twenty mg of a sample is dissolved in 15 ml of decalin, and the specific viscosity (ηsp) is measured in an atmosphere of 135°C using an Ubbelohde viscometer. After 5 ml of decalin is further added to this decalin solution for dilution, the specific viscosity is measured in the same manner. The dilution operation and the viscosity measurement are further repeated twice to provide measurement results, and the "ηsp/C" value in extrapolation of the concentration (: C) to zero based on the measurement results is defined as the intrinsic viscosity [η].

The content rate of the functional group structural unit of the olefin-based polymer [III] is 0.3 to 5.0% by mass, preferably 0.4 to 4.0% by mass. If the content rate of the functional group structural unit is too low, not only the polyamide [I] and the ethylene-α-olefin-unconjugated polyene copolymer rubber [II] are reduced in dispersibility and the design surface extruded is remarkably deteriorated, but also a reduction in mechanical strength may be caused. On the other hand, if the content rate of the functional group structural unit is too high, an abnormal reaction with the polyamide [I] occurs to cause gelation, and therefore melt fluidity may be reduced to result in a reduction in moldability.

The content rate of the functional group structural unit is the proportion of the mass (% by mass) of the compound having a functional group to 100% by mass of only the polymer of the monomer portion having no functional group of

the olefin-based polymer [III].

The content rate of the functional group structural unit of the olefin-based polymer [III] can be specified by the charging ratio of the compound having a functional group to the olefin-based polymer before modification when reacting them, or known means such as $^{13}C$-NMR measurement and $^{1}H$-NMR measurement. Specific NMR measurement conditions can include the following conditions.

In the case of $^{1}H$-NMR measurement, the measurement is performed using an ECX400 type nuclear magnetic resonance apparatus manufactured by JEOL Ltd. under conditions of deuterated orthodichlorobenzene as a solvent, a sample concentration of 20 mg/0.6 mL, a measurement temperature of 120°C, an observation nucleus of 1H (400 MHz), a sequence of a single pulse, a pulse width of 5.12 μs (45° pulse), a repetition time of 7.0 sec and a cumulative number of 500 or more. The reference chemical shift can be defined by setting hydrogen of tetramethylsilane at 0 ppm, and for example, the same result can also be obtained by setting the peak derived from residual hydrogen of deuterated orthodichlorobenzene at 7.10 ppm as the reference value of the chemical shift. A peak of 1H derived from a functional group-containing compound can be assigned by an ordinary method.

In the case of $^{13}C$-NMR measurement, the measurement is performed using an ECP500 type nuclear magnetic resonance apparatus manufactured by JEOL Ltd. as the measurement apparatus under conditions of a mixed solvent of orthodichlorobenzene/deuterated benzene (80/20% by vol) as a solvent, a measurement temperature of 120°C, an observation nucleus of 13C (125 MHz), single pulse proton decoupling, a pulse of 45°, a repetition time of 5.5 sec, a cumulative number of 10000 or more and a reference value of the chemical shift of 27.50 ppm. Assignment of various signals can be performed based on an ordinary method, and qualitative analysis can be performed based on the integrated value of signal strength.

The method for simply measuring the content rate of the functional group structural unit of the olefin-based polymer [III] also includes the following procedure. The functional group content rate of a polymer having a different functional group content rate is determined by NMR measurement, and the polymer whose functional group content rate is determined is subjected to infrared spectroscopic (IR) measurement. The calibration curve between the peak strength ratio of specific peaks in the infrared spectroscopic (IR) spectrum and the functional group content rate is created. The functional group content rate of any polymer is determined based on the calibration curve. While such a method is a simple method as compared with the above NMR measurement, respective corresponding calibration curves are required to be creased depending on the kinds of the base resin and the functional group. For such a reason, this method is a method preferably used in, for example, process management of resin production in a commercial plant.

Examples of an olefin-based polymer [III] available as a commercial product include Tafmer series (such as maleic anhydride modified ethylene-propylene rubber, maleic anhydride modified ethylene-butene rubber) and Admer (maleic anhydride modified polypropylene and maleic anhydride modified polyethylene) produced by Mitsui Chemicals, Inc.; Kuraprene (maleic anhydride modified isoprene rubber and maleic acid monomethyl ester modified isoprene rubber) and Septon (maleic anhydride modified SEPS) produced by Kuraray Co., Ltd.; Nucrel (ethylene-methacrylic acid copolymer) and HPR(maleic anhydride modified EEA and maleic anhydride modified EVA) produced by Du Pont-Mitsui Polychemicals; Royaltuf (maleic anhydride modified EPDM) produced by Chemtura Corporation; Kraton FG (maleic anhydride modified SEBS) produced by Kraton Performance Polymers Inc.; Nisseki polybutene (maleic anhydride modified polybutene) produced by JX Nippon Oil & Energy Corporation; Bondine (maleic anhydride modified EEA) produced by Arkema; Tuftec M (maleic anhydride modified SEBS) produced by Asahi Kasei Chemicals Corporation; Rexperal ET (maleic anhydride modified EEA) produced by Japan Polyethylene Corporation; Modic (maleic anhydride modified EVA, maleic anhydride modified polypropylene and maleic anhydride modified polyethylene) produced by Mitsubishi Chemical Corporation; Bondfast (E-GMA) produced by Sumitomo Chemical Co., Ltd.; Krynac (carboxy modified nitrile rubber) produced by LANXESS; and Auroren (maleic anhydride modified EEA) produced by Nippon Paper Industries Co., Ltd. (all the above are trade names). These can also be used in combinations of two or more.

<Rubber composition [X]>

The rubber composition [X] for use in the present invention is a composition comprising the polyamide [I], the ethylene-α-olefin-unconjugated polyene copolymer rubber [II] and the olefin-based polymer [III] described above.

The mass ratio ([II]/[III]) of the copolymer rubber [II] to the olefin-based polymer [III] in the rubber composition is preferably 95/5 to 60/40, more preferably 90/10 to 65/35, particularly preferably 90/10 to 70/30.

<Crosslinking agent [IV]>

The crosslinking agent [IV] for use in the present invention is a phenol resin-based crosslinking agent. Any crosslinking agent that can be dynamically crosslinked with the rubber composition can be used in combination with the phenol resin-based crosslinking agent as long as the effect of the present invention is not impaired, and for example, a sulfur-based crosslinking agent can be used.

The phenol resin-based crosslinking agent is typically a resol resin obtained by condensing an alkyl-substituted or unsubstituted phenol with an aldehyde (preferably formaldehyde) in the presence of an alkali catalyst. The alkyl group in the alkyl-substituted phenol is preferably an alkyl group having 1 to about 10 carbon atoms. Furthermore, dimethylol phenols or a phenol resin, substituted with an alkyl group having 1 to about 10 carbon atoms at the p-position, is preferable.

Crosslinking of a thermoplastic vulcanized rubber with the phenol resin-based crosslinking agent is described in, for example, US Patent No. 4311628, US Patent No. 2972600 and US Patent No. 3287440. Such a technique can also be used in the present invention.

Preferable examples of the phenol resin-based crosslinking agent include a compound represented by the following formula [IV-1].

Formula 2

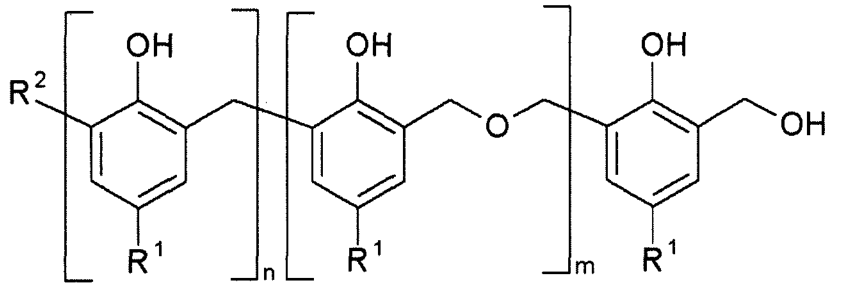

[IV-1]

(wherein n and m represent an integer of 0 to 20, $R^1$ represents an organic group such as an alkyl group, and $R^2$ represents -H or $-CH_2-OH$.)

In the formula [IV-1], n and m preferably represent an integer of 0 to 15, more preferably an integer of 0 to 10. $R^1$ preferably represents an organic group having less than 20 carbon atoms, more preferably an organic group having 4 to 12 carbon atoms.

As the phenol resin-based crosslinking agent, for example, an alkylphenol formaldehyde resin, a methylolated alkylphenol resin or a halogenated alkylphenol resin can be used. Among them, a halogenated alkylphenol resin is preferable. The halogenated alkylphenol resin is an alkylphenol resin in which a hydroxyl group at the molecular chain terminal is substituted with a halogen atom such as bromine, and examples thereof include a compound represented by the following formula [IV-2].

Formula 3

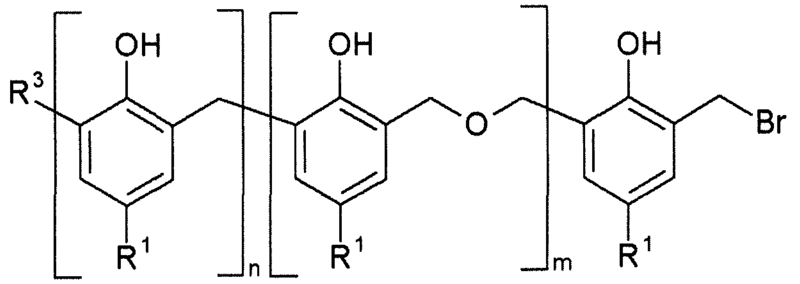

[IV-2]

(wherein n and m represent an integer of 0 to 20, $R^1$ represents an organic group such as an alkyl group, and $R^3$ represents -H, $-CH_3$ or $-CH_2-Br$.)

In the formula [IV-2], n and m preferably represent an integer of 0 to 15, more preferably an integer of 0 to 10. $R^1$ preferably represents an organic group having less than 20 carbon atoms, more preferably an organic group having 4 to 12 carbon atoms.

The phenol resin-based crosslinking agent described above is available as a commercial product. Examples of such a commercial product include Tackirol 201, Tackirol 250-1 and Tackirol 250-III produced by Taoka Chemical Co., Ltd.; SP1045, SP1055 and SP1056 produced by SI Group Inc.; Shonol CRM produced by Showa Denko K. K.; Tamanol 531 produced by Arakawa Chemical Industries, Ltd.; Sumilite Resin PR produced by Sumitomo Bakelite Co., Ltd.; and Resitop produced by Gunei Chemical Industry Co., Ltd. (all the above are trade names). These can also be used in combinations of two or more. Among them, Tackirol 250-III (brominated alkylphenol formaldehyde resin) produced

by Taoka Chemical Co., Ltd. and SP1055 (brominated alkylphenol formaldehyde resin) produced by SI Group Inc. are preferable.

When the crosslinking agent [IV] is a powdery crosslinking agent, the average particle size thereof is preferably 0.1 μm to 3 mm, more preferably 1 μm to 1 mm, particularly preferably 5 μm to 0.5 mm. A flake-shaped curing agent is preferably used after being formed into a powder by a pulverizer such as a jet mill or a pulverizer equipped with a pulverizing blade.

Herein, when organic peroxide is used as the crosslinking agent [IV], a suitable melt kneading temperature for the elastomer composition of the present invention is relatively high and therefore the decomposition speed is too high in the case of organic peroxide. As a result, the crosslinking reaction of the rubber components ([II], [III]) rapidly progresses, and sufficient kneading with the polyamide [I] component cannot be made. In addition, dispersion is insufficient to thereby cause physical properties of the polyamide-based thermoplastic elastomer composition to be remarkably deteriorated.

<Dynamic crosslinking of polyamide-based thermoplastic elastomer composition [Y]>

The polyamide-based thermoplastic elastomer composition [Y] is a resin composition in which the rubber composition and the crosslinking agent [IV] are dynamically crosslinked.

Specifically, the rubber composition [X] and the crosslinking agent [IV] are crosslinked in the melt flow state (dynamic state) to thereby provide the polyamide-based thermoplastic elastomer composition [Y]. Such a dynamic crosslinking reaction is usually performed by supplying the rubber composition [X] and the crosslinking agent [IV] to a melt kneading apparatus, and heating them to a predetermined temperature for melt kneading.

Specific examples of the melt kneading apparatus include a twin screw extruder, a single screw extruder, a kneader and a Banbury mixer. Among them, a twin screw extruder is preferable in terms of a shear force and continuous productivity.

The melt kneading temperature is usually 200 to 320°C. The melt kneading time is usually 0.5 to 30 minutes.

Such dynamic crosslinking allows a dynamically crosslinked body to be formed in the polyamide-based thermoplastic elastomer composition [Y], resulting in formation of a sea-island structure. The sea phase (matrix component) is configured from the polyamide and serves as a phase exhibiting thermoplasticity. On the other hand, the island phase (dispersion component) is configured from the rubber component crosslinked and serves as a phase exhibiting rubber elasticity.

That is, in the polyamide-based thermoplastic elastomer composition [Y], the polyamide [I] is a matrix component, the dispersion component dispersed in the matrix component is a particle including the copolymer rubber [II] and the olefin-based polymer [III], and the following (1) is satisfied:

(1) a cross sectional area of the particle, in which the particle size is 5 μm or more, of the dispersion component is measured, and the proportion of the cumulative cross sectional area of the area of the particle to the entire cross sectional area analyzed is 10% or less.

The proportion of the total cross sectional area of the particle having a particle size of 5.0 μm or more in the entire cross sectional area is preferably 5.0% or less, and the proportion of the total cross sectional area of the particle having a particle size of 5.0 μm or more in the entire cross sectional area is further preferably 2.5% or less. Extremely preferable is a case where the total cross sectional area of the particle having a particle size of 5.0 μm or more in the entire cross sectional area is absent at all.

The average particle size of the dispersion component is 0.5 μm or more and 5.0 μm or less, preferably 0.5 μm or more and 4.0 μm or less, further preferably 0.5 μm or more and 2.5 μm or less.

When the proportion of the cumulative cross sectional area of the area of the particle to the entire cross sectional area analyzed as described above is in the range of 10% or less, not only the fluidity of the entire composition is uniform and there is suppressed attachment of a trace of the composition onto the edge of a discharge port of an extrusion die in accordance with pulsation in extrusion, so-called development of die drool, but also the flow rate (fluidity) at each of a portion in contact with a metal inner wall and a portion in no contact therewith is stabilized to inhibit the resin (in particular, rubber dispersion component) from being retained on the metal inner wall, resulting in remarkable suppression of development of die drool as a whole.

Control of the particle size of the dispersion component in the present invention is performed by, for example, the order of compounding, the kneading temperature, the speed of screw rotations and screw arrangement.

In order to provide a small particle, it is preferable to achieve a high shear force and to suppress the crosslinking speed. Specifically, this is achieved by a screw arrangement assembled so that high distributive mixing is made at the front half of a kneading portion of the extruder under low shear and high shear is achieved at the rear half thereof. A condition, where high temperature and high shear are caused at the front half of the kneading portion even if a screw segment is appropriately designed, causes aggregation of the rubber component crosslinked, not to allow the requirement

where the proportion of the cumulative cross sectional area is 10% or less to be satisfied.

The sheet-shaped or tubular molded body thus obtained is configured from a composition having a phase structure where the morphology of particle (dispersion phase) of the crosslinked rubber component including the copolymer rubber [II] and the olefin-based polymer [III] / polyamide resin (matrix phase) of the present embodiment is controlled and the polyamide-based thermoplastic elastomer composition is finely dispersed in the matrix phase, and therefore has, as characteristics thereof, suppression of die drool, pressure resistance, and creep resistance performance.

TEM measurement

Any cross section of each test piece subjected to extrusion molding as described above was analyzed in the range of about 45 μm × 75 μm or more by using a transmission electron microscope (measurement apparatus: H-7650 manufactured by Hitachi High-Technologies Corporation) (at a magnification of 3000).

The analysis was made by a binarization process using image analysis software ImageJ. In this image (Figure 1), the occupation region of each particle of the polyamide resin (Figure 1, white portion as compared with other portions, matrix), the ethylene-α-olefin-unconjugated polyene copolymer rubber [II] including the structural unit derived from the unconjugated polyene [c] having at least one carbon-carbon double bond in one molecule, and the olefin-based polymer [III] was identified.

The area of the occupation region of each particle identified was calculated by image analysis.

Then, the diameter of a true circle having an area equal to the above area was determined, and the arithmetic average of the values determined with respect to the respective occupation regions was defined as the average particle size measured of each particle.

That is, the particle size of each particle is defined by $(4S/\pi)^{0.5}$ using the area S determined of each particle.

Herein, the average particle size in the present embodiment is measured with respect to a particle size of 0.5 μm or more. The particle having a particle size of 0.5 μm or more is classified to a particle group (A).

The reason is considered as follows: a particle having a particle size of less than 0.5 μm (classified to a particle group (B)) has an area ratio of less than 1% in the entire particle, a particle group of an external additive, independently present, is also included in a large amount, and therefore there is no influence on suppression of die drool, pressure resistance, and the effect of creep resistance in the present embodiment.

Exemplary Embodiment 1: Polyamide-based thermoplastic elastomer composition [Y1]

A polyamide-based thermoplastic elastomer composition [Y1] according to the present Exemplary Embodiment is a composition in which, in 100% by mass of the total of the components [I], [II], [III] and [IV] in the polyamide-based thermoplastic elastomer composition [Y], the rubber composition [X] containing 10 to 60% by mass of the polyamide [I], 30 to 86% by mass of the ethylene-α-olefin-unconjugated polyene copolymer rubber [II] and 3 to 30% by mass of the olefin-based polymer [III], and 1 to 10% by mass of the phenol resin-based crosslinking agent [IV] are dynamically crosslinked.

The proportion of the polyamide [I] is 10 to 60% by mass, preferably 15 to 50% by mass, more preferably 20 to 45% by mass. The proportion of the copolymer rubber [II] is 30 to 86% by mass, preferably 33 to 80% by mass, more preferably 33 to 70% by mass. The proportion of the olefin-based polymer [III] is 3 to 30% by mass, preferably 5 to 20% by mass, more preferably 5 to 15% by mass. The proportion of the crosslinking agent [IV] is 1 to 10% by mass, preferably 1 to 8% by mass, more preferably 2 to 6% by mass.

Various addition components other than the above-described components may also be if necessary added to the polyamide-based thermoplastic elastomer composition [Y1] of the present invention as long as the object of the present invention is not impaired. Examples of the addition components include a crosslinking aid, a plasticizer, an inorganic filler, a lubricant, a light stabilizer, a pigment, a flame retardant, an antistatic agent, a silicone oil, an anti-blocking agent, an ultraviolet absorber and an antioxidant.

<Molded article>

A molded article of the present Exemplary Embodiment is a molded article obtained from the polyamide-based thermoplastic elastomer composition [Y1]. The application thereof is not particularly limited. For example, the molded article is very useful as molded articles for various applications, such as an automobile component, a building material component, sporting equipment, a medical equipment component and an industrial component.

Exemplary Embodiment 2: Polyamide-based thermoplastic elastomer composition [Y2]

A polyamide-based thermoplastic elastomer composition [Y2] according to the present Exemplary Embodiment

is a composition in which, in 100% by mass of the total of the components [I], [II], [III] and [IV] in the polyamide-based thermoplastic elastomer composition [Y], the rubber composition [X] containing 30 to 87.7% by mass of the polyamide [I], 10 to 45% by mass of the ethylene-$\alpha$-olefin-unconjugated polyene copolymer rubber [II] and 2 to 20% by mass of the olefin-based polymer [III], and 0.3 to 5.0% by mass of the phenol resin-based crosslinking agent [IV] are dynamically crosslinked.

The proportion of the polyamide [I] is 30 to 87.7% by mass, preferably 40 to 86% by mass, more preferably 45 to 85% by mass. The proportion of the copolymer rubber [II] is 10 to 45% by mass, preferably 11 to 43% by mass, more preferably 11 to 42% by mass. The proportion of the olefin-based polymer [III] is 2 to 20% by mass, preferably 3 to 15% by mass, more preferably 3 to 12% by mass. The proportion of the crosslinking agent [IV] is 0.3 to 5.0% by mass, preferably 0.5 to 4.0% by mass, more preferably 1.0 to 4.0% by mass.

Various addition components other than the above-described components may also be if necessary added to the polyamide-based thermoplastic elastomer composition [Y2] according to the present Exemplary Embodiment as long as the object of the present invention is not impaired. Examples of the addition components include a crosslinking aid, an inorganic filler, a lubricant, a light stabilizer, a pigment, a flame retardant, antistatic agent, anti-blocking agent, an ultraviolet absorber and an antioxidant.

Herein, it is preferable that a plasticizer such as butylbenzenesulfonamide (BBSA) be not added to the polyamide-based thermoplastic elastomer composition [Y2] according to the present Exemplary Embodiment or such a plasticizer be added in a small amount. Specifically, the amount of the plasticizer to be added to 100 parts by mass of the resin is preferably 0 to 5 parts by mass, more preferably 0 to 3 parts by mass.

<Industrial tube>

The industrial tube of the present invention is an industrial tube having at least a layer including the polyamide-based thermoplastic elastomer composition [Y2] according to the present Exemplary Embodiment. The industrial tube means a tube particularly used for industrial equipment. Specific examples include a tube through which a fluid (such as fuel, solvent, chemical agent, gas) required for industrial equipment such as a vehicle (e.g., automobile), pneumatic-hydraulic equipment, painting equipment and medical equipment passes. In particular, the tube is very useful in applications such as a tube for vehicle piping (e.g., fuel system tube, inlet system tube and cooling system tube), a pneumatic tube, a hydraulic tube, a paint spray tube and a medical tube (e.g., catheter).

The outer diameter of the industrial tube is preferably 2 mm to 50 mm, more preferably 6 mm to 30 mm. The thickness is preferably 0.2 mm to 10 mm, more preferably 0.5 to 7 mm.

The industrial tube may be a monolayer tube or may be a multi-layer tube such as a bilayer tube or a trilayer tube. The monolayer tube is a tube configured from only one composition including at least the thermoplastic elastomer composition [Y2] according to the present Exemplary Embodiment. The multi-layer tube is, for example, a tube having a laminate structure including a layer of the polyamide-based thermoplastic elastomer composition [Y2] according to the present Exemplary Embodiment, and one or more layers (other layers) other than the above layer. The layer of the polyamide-based thermoplastic elastomer composition [Y2] according to the present Exemplary Embodiment has sufficient barrier properties and flexibility in combination, and therefore is not required to be formed into a multi-layer structure unlike a conventional barrier layer and is very advantageous in terms of production cost.

A material constituting other layers of the multi-layer tube may be appropriately determined, if necessary. Examples of the material include at least one resin selected from the group consisting of a fluororesin, a high density polyethylene resin, a polybutylene naphthalate resin (PBN), an aliphatic polyamide resin, an aromatic polyamide resin, a metaxylene group-containing polyamide resin, an ethylene-vinyl acetate copolymer saponified product (EVOH) and a polyphenylene sulfide resin (PPS). An adhesion layer may also be disposed for the purpose of an enhancement in adhesiveness between respective layers.

Specific examples of the fluororesin include polytetrafluoroethylene (PTEF), polyvinylidene fluoride (PVDF) and polyvinyl fluoride (PVF). The fluororesin may also be a resin partially including chlorine, such as polychlorofluoroethylene (PCTFE), or a copolymer with ethylene.

<Exemplary Embodiment 3: Polyamide-based thermoplastic elastomer composition [Z]>

A polyamide-based thermoplastic elastomer composition [Z] according to the present Exemplary Embodiment includes a polyamide [Z1] and a polyamide-based resin composition [Z2] described below. In the polyamide-based thermoplastic elastomer composition [Z] according to the present Exemplary Embodiment, the polyamide [Z1] serves as a high crystalline component, and the polyamide-based resin composition [Z2] serves as a flexible component.

<Polyamide [Z1]>

The polyamide [Z1] for use in the present Exemplary Embodiment is a polyamide having a melting point (Tm) determined by differential scanning calorimetry (DSC) of 210 to 270°C and an amount of melting heat (ΔH) determined by DSC of 45 to 80 mJ/mg.

Specific examples of the polyamide [Z1] include polycaproamide (nylon 6), polytetramethylene adipamide (nylon 46), polyhexamethylene adipamide (nylon 66), polyundecamethylene adipamide (nylon 116), polymetaxylylene adipamide (nylon MXD6), polyparaxylylene adipamide (nylon PXD6), polytetramethylene sebacamide (nylon 410), polyhexamethylene sebacamide (nylon 610), polydecamethylene adipamide (nylon 106), polydecamethylene sebacamide (nylon 1010), polyhexamethylene dodecanamide (nylon 612), polydecamethylene dodecanamide (nylon 1012), polyhexamethylene isophthalamide (nylon 6I), polytetramethylene terephthalamide (nylon 4T), polypentamethylene terephthalamide (nylon 5T), poly2-methylpentamethylene terephthalamide (nylon M-5T), polyhexamethylene terephthalamide (nylon 6T), polyhexamethylene hexahydroterephthalamide (nylon 6T(H)), polynonamethylene terephthalamide (nylon 9T), polyundecamethylene terephthalamide (nylon 11T), polydodecamethylene terephthalamide (nylon 12T), polybis(3-methyl-4-aminohexyl)methane terephthalamide (nylon PACMT), polybis(3-methyl-4-aminohexyl)methane isophthalamide (nylon PACMI), polybis(3-methyl-4-aminohexyl)methane dodecanamide (nylon PACM12) and polybis(3-methyl-4-aminohexyl)methane tetradecanamide (nylon PACM14). In particular, polydodecamethylene terephthalamide (nylon 12T), polydecamethylene sebacamide (nylon 1010) and polydecamethylene dodecamide (nylon 1012) are preferable in terms of processability, low water absorbability and impact resistance.

The melting point (Tm) determined by differential scanning calorimetry (DSC) of the polyamide [Z1] is 210 to 270°C, preferably 215 to 265°C, more preferably 220 to 260°C. The polyamide [Z1] has such a melting point (Tm) to thereby exhibit excellent heat resistance. This melting point (Tm) is measured under the following conditions. First, the polyamide [Z1] is heated and held once at 320°C for 5 minutes, and then cooled to 23°C at a rate of 10°C/min and thereafter heated at a rate of 10°C/min. The endothermic peak based on fusion here corresponds to the melting point (Tm) of the polyamide.

The amount of melting heat (ΔH) of the polyamide [Z1], determined by DSC, is 45 to 80 mJ/mg, preferably 45 to 75 mJ/mg, more preferably 50 to 70 mJ/mg. When the polyamide [Z1] has such an amount of thermal fusion, the polyamide [Z1] has a proper crystallinity and thus exhibits excellent high temperature rigidity. This amount of melting heat (ΔH) is measured under the following conditions. First, the polyamide [Z1] is heated and held once at 320°C for 5 minutes, and then cooled to 23°C at a rate of 10°C/min and thereafter heated at a rate of 10°C/min. The amount of heat of fusion was calculated from the integrated value of the endotherm peak based on fusion here.

The polyamide-based thermoplastic elastomer composition [Z] according to the present Exemplary Embodiment is a composition including the above-described polyamide [Z1] and the polyamide-based thermoplastic elastomer composition [Y2] as the polyamide-based resin composition [Z2]. In 100% by mass of the total of the components [Z1] and [Z2] in the polyamide-based thermoplastic elastomer composition, the proportion of the polyamide [Z1] is 10 to 35% by mass, preferably 15 to 35% by mass, more preferably 15 to 30% by mass. The proportion of the polyamide-based resin composition [Z2] is 65 to 90% by mass, preferably 65 to 85% by mass, more preferably 70 to 85% by mass.

The polyamide-based thermoplastic elastomer composition [Z] of the present invention is obtained by mixing the polyamide [Z1] and the polyamide-based resin composition [Z2] (polyamide-based thermoplastic elastomer composition [Y2]). The mixing method is not particularly limited, and such mixing may be made using a known apparatus. For example, a melt kneading apparatus such as a twin screw extruder, a single screw extruder, a kneader or a Banbury mixer, recited above, can be used.

Various addition components other than the above-described components may also be if necessary added to the polyamide-based thermoplastic elastomer composition [Z] of the present invention as long as the object of the present invention is not impaired. Examples of the addition components include a crosslinking aid, a plasticizer, an inorganic filler, a lubricant, a light stabilizer, a pigment, a flame retardant, an antistatic agent, a silicone oil, an anti-blocking agent, an ultraviolet absorber and an antioxidant.

According to the present embodiment, a specific high crystalline component (polyamide [Z1]) and a specific flexible component (polyamide-based resin composition [Z2] = polyamide-based thermoplastic elastomer composition [Y2]) are compounded in a specific ratio to thereby provide a polyamide-based thermoplastic elastomer composition [Z] having good elasticity retention rate at a high temperature, oil resistance and moldability in combination, and a molded article thereof.

While the reason why such good characteristics are exhibited is not necessarily clear, it is considered that the high crystalline component is finely dispersed in the composition to thereby form a domain that is strong and is not fused even in a high temperature range, and the domain serves as a pseudo network to suppress a reduction in elastic modulus in a high temperature range and to keep a proper viscosity in melting of the resin. Then, such characteristics are exhibited to thereby enable to favorably perform a particular molding method (such as blow molding) in which a molding material is required to have a high elasticity retention rate at a high temperature. Accordingly, the polyamide-based thermoplastic

elastomer composition [Z] according to the present Exemplary Embodiment is very useful as a material for various molded articles produced by such a molding method, such as a resin flexible boot such as an automobile constant velocity joint boot.

<Molded article>

A molded article of the present Exemplary Embodiment is a molded article obtained from the polyamide-based thermoplastic elastomer composition [Z] according to the present Exemplary Embodiment by injection molding or blow molding. In particular, the polyamide-based thermoplastic elastomer composition [Z] according to the present Exemplary Embodiment has good elasticity retention rate at a high temperature, oil resistance and moldability in combination, and therefore can be widely utilized in various applications (e.g., automobile and electrical product) where such physical properties are required. Specific examples of the molded article of the present Exemplary Embodiment include a boot component such as a constant velocity joint boot, an oil seal, a gasket, packing, a dust cover, a valve, a stopper, a precision seal rubber, and a weather strip. Among them, an automobile constant velocity joint boot including the polyamide-based thermoplastic elastomer composition [Z] according to the present Exemplary Embodiment is preferable.

As the method for producing the automobile constant velocity joint boot, for example, a known method such as an injection molding method or a blow molding method (injection blow molding method or press blow molding method) can be used. The bellows shape of the boot is not particularly limited, and usually has 2 ridges/2 valleys to 15 ridges/15 valleys, preferably 3 ridges/3 valleys to 8 ridges/8 valleys.

## Examples

Hereinafter, the present invention is more specifically described with reference to Examples.

First, production examples of polyamides used are described.

Polyamide [I-1]: (6T6I66 = 50/35/15)
Dicarboxylic acid component = terephthalic acid: 50% by mass, isophthalic acid: 35% by mass, adipic acid: 15% by mass
Diamine component = 1,6-hexanediamine
Melting point (Tm) = 276°C
Proportion of end group terminated by end-terminating agent (benzoic acid) = 79%
Amount of terminal amino group in molecular chain = 21 mmol/kg

Polyamide [1-1] was synthesized as follows. An autoclave having an inner volume of 13.6 L was charged with 1986 g (12.0 mol) of terephthalic acid, 2800 g (24.1 mol) of 1,6-hexanediamine, 1390 g (8.4 mol) of isophthalic acid, 524 g (3.6 mol) of adipic acid, 36.5 g (0.3 mol) of benzoic acid, 5.7 g (0.08% by mass relative to raw materials) of sodium hypophosphite monohydrate and 545 g of distilled water, and purged with nitrogen. Stirring was initiated from 190°C, and the internal temperature was raised to 250°C over 3 hours. Here, the internal pressure of the autoclave was increased to 3.03 MPa. After the reaction was continued for 1 hour as it was, release to the atmosphere was made through a spray nozzle disposed at the lower portion of the autoclave to take out a low condensate. Thereafter, the low condensate was cooled to room temperature, pulverized by a pulverizer so as to have a particle size of 1.5 mm or less, and dried at 110°C for 24 hours. The amount of water in the resulting low condensate was 4100 ppm, and the intrinsic viscosity [η] of the low condensate was 0.14 dl/g. Next, this low condensate was placed in a tray-type solid phase polymerization apparatus, purged with nitrogen, and heated to 180°C over about 1 hour and 30 minutes. Thereafter, the reaction was performed for 1 hour and 30 minutes, and the temperature was decreased to room temperature. The intrinsic viscosity [η] of the resulting polyamide was 0.18 dl/g. Thereafter, melt polymerization was performed in a twin screw extruder having a screw diameter of 30 mm and L/D = 36 at a barrel set temperature of 340°C, a screw rotation number of 200 rpm and a resin feed speed of 5 kg/h, to provide polyamide [1-1] above.

Polyamide [1-2]: (6T6I66 = 44/36/20)
Dicarboxylic acid component = terephthalic acid: 44% by mass, isophthalic acid: 36% by mass, adipic acid: 20% by mass
Diamine component = 1,6-hexanediamine
Melting point (Tm) = 264°C
Proportion of end group terminated by end-terminating agent (benzoic acid) = 80%
Amount of terminal amino group in molecular chain = 19mmol/kg

Polyamide [1-2] was obtained in the same manner as in polyamide [1-1] except that the amounts of terephthalic acid, isophthalic acid and adipic acid were changed.

Polyamide [1-3]: (9T8T = 50/50)
Dicarboxylic acid component = terephthalic acid
Amine component = 1,9-nonanediamine: 50% by mass, 2-methyl-1,8-octanediamine: 50% by mass
Melting point (Tm) = 265°C
Proportion of end group terminated by end-terminating agent (benzoic acid): 76%

Amount of terminal amino group in molecular chain = 18 mmol/kg

This polyamide [1-3] was obtained in the same manner as in polyamide [1-1] except that the raw materials used were changed to 3971 g (23.9 mol) of terephthalic acid, 1899 g (12.0 mol) of 1,9-nonanediamine, 1900 g (12.1 mol) of 2-methyl-1,8-octanediamine, 36.5 g (0.3 mol) of benzoic acid, 5.7 g (0.08% by mass relative to raw materials) of sodium hypophosphite monohydrate and 530 g of distilled water.

Polyamide [1-4]: (10T)
Dicarboxylic acid component = terephthalic acid
Diamine component = decamethylenediamine
Aminocarboxylic acid component = 11-aminoundecanoic acid
Melting point (Tm) = 260°C
Proportion of end group terminated by end-terminating agent (benzoic acid) = 81%
Amount of terminal amino group in molecular chain: 17 mmol/kg

This polyamide [1-4] was obtained in the same manner as in polyamide [1-1] except that the raw materials used were changed to 2391 g (14.4 mol) of terephthalic acid, 2478 g (14.4 mol) of decamethylenediamine, 1929 g (9.6 mol) of 11-aminoundecanoic acid, 36.5 g (0.3 mol) of benzoic acid, 5.7 g (0.08% by weight relative to raw materials) of sodium hypophosphite-hydrate and 530 g of distilled water.

Polyamide [1-5]: (6T6I66 = 65/20/15)
Dicarboxylic acid component = terephthalic acid: 65% by mass, isophthalic acid: 20% by mass, adipic acid: 15% by mass
Diamine component = hexamethylenediamine
Melting point (Tm) = 295°C
Proportion of end group terminated by end-terminating agent = 79%
Amount of terminal amino group in molecular chain: 23 mmol/kg

This polyamide [1-5] was obtained in the same manner as in polyamide [1-1] except that the amounts of terephthalic acid, isophthalic acid and adipic acid were changed.

<Example A: Polyamide-based thermoplastic elastomer composition [Y1]>

<Example A1>

Polyamide [I-1] was used as the polyamide [I].

An ethylene/propylene/5-ethylidene-2-norbornene copolymer rubber ([η] = 2.4 dl/g, ethylene content: 65% by mass, diene content: 4.6% by mass) was used as the copolymer rubber [II].

A modified polyolefin (maleic anhydride modified ethylene-1-butene copolymer, amount of maleic anhydride grafting modification (content rate of functional group structural unit): 0.97% by mass, intrinsic viscosity [η] measured in decalin solution at 135°C: 1.98 dl/g), synthesized as follow, was used as olefin-based polymer [III-1].

First, a glass flask sufficiently purged with nitrogen was charged with 0.63 mg of bis(1,3-dimethylcyclopentadienyl)zirconium dichloride, and 1.57 ml of a solution of methylaluminoxane in toluene (Al; 0.13 mmol/l) and 2.43 ml of toluene were further added to thereby provide a catalyst solution. To a stainless autoclave having an inner volume of 2 L, sufficiently purged with nitrogen, 912 ml of hexane and 320 ml of 1-butene were charged, and the temperature in the system was raised to 80°C. Subsequently, 0.9 mmol of triisobutylaluminum and 2.0 ml of the above catalyst solution (0.0005 mmol as Zr) were loaded under pressure by ethylene to initiate polymerization. Ethylene was continuously fed to thereby keep the total pressure at 8.0 kg/cm$^2$-G, and the polymerization was performed at 80°C for 30 minutes. A small amount of ethanol was introduced into the system to stop the polymerization, and thereafter unreacted ethylene was purged from the system. The resulting solution was poured into a large excess of methanol to thereby allow a white solid to be precipitated. This white solid was collected by filtration, and dried under reduced pressure overnight to provide ethylene-1 -butene copolymer as a white solid. This ethylene-1-butene copolymer had the density of 0.862 g/cm$^3$, the MFR (ASTM D1238 standard, 190°C, load: 2160 g) of 0.5 g/10 min, and the content rate of a 1-butene structural unit of 4% by mole. This ethylene-1-butene copolymer (100 parts by mass) was mixed with 1.0 part by mass of maleic anhydride and 0.04 parts by mass of peroxide (produced by NOF Corporation, trade name: Perhexyne 25B), and the resulting mixture was subjected to melt grafting modification in a single screw extruder set at 230°C, to thereby provide the maleic anhydride modified ethylene-1-butene copolymer.

A powder obtained by agitating a flake-shaped brominated alkylphenol formaldehyde resin (produced by Taoka Chemical Co., Ltd., trade name: Tackirol 250-III) by a Henschel mixer for 10 seconds was prepared as the crosslinking agent [IV].

Then, 40% by mass of polyamide [I-1], 45% by mass of the copolymer rubber [II], 12% by mass of olefin-based polymer [III-1], 3% by mass of the crosslinking agent [IV] and a small amount of a crosslinking aid (manufactured by Hakusuitech Co., Ltd., zinc oxide JIS#2) were pre-mixed, and the resultant was fed to a twin screw extruder (manufactured by Japan Steel Works, LTD., TEX-30) and melt kneaded at a cylinder temperature of 280°C and a screw rotation number

of 300 rpm. A strand extruded from this twin screw extruder was cut to provide pellets of a polyamide-based thermoplastic elastomer composition.

The pellets thus obtained were used to perform the evaluation tests of respective physical properties. The results are shown in Table 1.

<Examples A2 to A12> (Examples A8 to A12 are comparative examples)

Respective pellets were produced in the same manner as in Example A1 except that the kinds of the polyamide [I] and the olefin-based polymer [III] used and the compounding ratio thereof were changed as shown in Table 1, and the evaluation tests were performed. The results are shown in Table 1. Olefin-based polymers [III-2] and [III-3] were as follows.

[Olefin polymer (III-2)]

Olefin polymer (III-2) was prepared in the same manner as in olefin polymer (III-1) except that the amount of maleic anhydride to be added in modification of the ethylene-1-butene copolymer before modification in production of olefin polymer (III-1) was changed to 0.5 parts by weight. The amount of maleic anhydride grafting modification was 0.50 wt.%. The intrinsic viscosity [η] measured in decalin solution at 135°C was 1.79 dl/g.

[Olefin polymer (III-3)]

The ethylene-1butene copolymer before modification in production of olefin polymer (III-1) was used as it was.

Table 1

| | | Example | | | | | | | | | | | |
|---|---|---|---|---|---|---|---|---|---|---|---|---|---|
| | | A1 | A2 | A3 | A4 | A5 | A6 | A7 | A8 | A9 | A10 | A11 | A12 |
| Polyamide [I] | Kind | I-1 | I-2 | I-2 | I-2 | I-3 | I-4 | I-2 | I-5 | I-2 | I-2 | I-3 | I-2 |
| | Melting point(°C) | 276 | 264 | 264 | | 265 | 260 | 264 | 295 | 264 | | 265 | 264 |
| | Amount (mass%) | 40 | 50 | 40 | 30 | 40 | 40 | 40 | 40 | 41 | 62 | 62 | 40 |
| Copolymer rubber[II] | Amount (mass%) | 45 | 35 | 45 | 55 | 45 | 45 | 45 | 45 | 46 | 23 | 23 | 45 |
| Olefin polymer [III] | Kind | III-1 | III-1 | III-1 | III-1 | III-1 | III-1 | III-2 | III-1 | III-1 | III-1 | III-1 | III-3 |
| | Amount (mass%) | 12 | 12 | 12 | 12 | 12 | 12 | 12 | 12 | 13 | 12 | 12 | 12 |
| Crosslinking agent [IV] | Amount (mass%) | 3 | 3 | 3 | 3 | 3 | 3 | 3 | 3 | - | 3 | 3 | 3 |
| Proportion of rubber component in composition (mass%) | | 57 | 47 | 57 | 67 | 57 | 57 | 57 | 57 | 59 | 35 | 35 | 35 |
| Tensile properties | 100% Modulus (MPa) | 6.1 | 6.9 | 5.8 | 4.5 | 5.9 | 5.5 | 5.8 | Not molded | 11.5 | - | - | - |
| | Breaking strength (MPa) | 11 | 10.9 | 12.1 | 9.5 | 9.1 | 8.9 | 10.7 | | 6.8 | 24 | 18 | 5.8 |
| | Elongation (%) | 250 | 240 | 260 | 300 | 290 | 310 | 250 | | 170 | 60 | 90 | 70 |
| Durometer A hardness | Shore-A hardness (degrees) | 85 | 89 | 83 | 72 | 84 | 83 | 84 | Not measured | 95 | >95 | >95 | >95 |
| Compression set (100°C×22h)(%) | | 59 | 59 | 55 | 49 | 48 | 49 | 56 | Not measured | 100 | 100 | 100 | 100 |
| State of design surface extruded | | A | A | A | A | A | A | A | C | C | A | A | C |
| * Examples A8 to A12 are comparative examples | | | | | | | | | | | | | |

Evaluation of physical properties in each Example was performed according to the following methods.

[Tensile properties]

A sheet sample of 20 cm × 20 cm × 2 mm, prepared from the pellets of the thermoplastic elastomer composition using a 50 t press machine (280°C), was used as a test piece, and subjected to a tensile test under conditions of a measurement temperature of 25°C and a tensile speed of 500 mm/min according to JIS6251 to measure the 100% tensile modulus (MPa), the strength at breaking TB (MPa) and the elongation EB (%).

[Durometer A hardness]

The same sheet sample as in the measurement of tensile physical properties was used as a test piece, and the JIS A hardness was measured by a durometer A hardness tester according to JIS 6253. Specifically, the test piece was stacked for three sheets, a load of 1.0 kg was applied thereto, and the standard hardness was read within one second after contacting the load on the pressing surface of the test piece and the read value was defined as the Shore-A hardness (degrees).

[Compression set]

The same sheet sample as in the measurement of tensile physical properties was used as a test piece, the test piece was stacked for six sheets to thereby prepare a block-shaped sample, and the sample was mounted to a mold for compression set measurement. The sample was compressed so that the height of the test piece was a quarter of the height of the test piece before loading, and set together with the mold in a gear oven at 100°C and heat-treated for 22 hours. Next, the test piece was taken out from the mold and cooled for 30 minutes, and thereafter the height of the test piece was measured to calculate the compression set [CS] (%) from the following calculation expression.

$$\text{Compression set [CS](\%)} = \{(t0 - t1)/(t0 - t2)\} \times 100$$

t0: Height of test piece before test.
t1: Height of test piece after heat treatment and cooling for 30 minutes.
t2: Height of test piece mounted to measurement mold.

[State of design surface extruded]

The pellets of the thermoplastic elastomer composition were extruded at 280°C in a nitrogen atmosphere using a single screw extruder having L/D = 30 and a screw diameter of 50 mm, and molded to a predetermined shape due to a mouthpiece having a width of 1.5 cm and a thickness of 2 mm. The state of the design surface extruded of the resulting plate specimen (1.5 cm in width × 2 mm in thickness × 2 m in length) was rated on a 3-point scale according to the following criteria.
"A": Smoothness was excellent and good outer appearance was exhibited.
"B": Slight irregularities were observed and surface gloss was poor.
"C": Many fine irregularities were observed and smoothness was poor.

<Evaluation>

It is found that in each of Examples A1 to A7, the 100% modulus (MPa) is properly low, the elongation (%) is large and also the break strength (MPa) is large, and therefore the tensile properties are excellent. In addition, it is found that the Shore-A hardness is a proper value and therefore the flexibility is also sufficient. Furthermore, the compression set is also small. Accordingly, it can be said that comprehensively excellent rubber elasticity is achieved in each of Examples A1 to A7. Moreover, the extrusion moldability is also good.
On the other hand, in Example A8, the melting point (Tm) of polyamide [1-5] used was too high to perform extrusion molding, and respective physical properties could not be measured. In Example A9, no crosslinking agent [IV] was used, and therefore the elongation (%) and the break strength (MPa) were small, the compression set was large, and the extrusion moldability was also poor. In each of Examples A10 and A11, the amount of the polyamide [I] was large and the amount of the copolymer rubber [II] was small, and therefore the elongation (%) was small and the compression set was large. In Example A12, unmodified olefin-based polymer [III-3] was used and therefore the elongation (%) was small and the compression set was large, and furthermore the state of the design surface extruded was also deteriorated.

<Example B: Polyamide-based thermoplastic elastomer composition [Y2]: Industrial tube>

<Example B1>

In the same manner as in Example A1, polyamide [1-1] as the polyamide [I]; an ethylene/propylene/5-ethylidene-2-norbornene copolymer rubber ([η] = 2.4 dl/g, ethylene content: 65% by mass, diene content: 4.6% by mass) as the copolymer rubber [II]; the modified polyolefin (maleic anhydride modified ethylene-1-butene copolymer [III-1], amount of maleic anhydride grafting modification (content rate of functional group structural unit): 0.97% by mass, intrinsic viscosity [η] measured in decalin solution at 135°C: 1.98 dl/g) synthesized in Example A1 as the olefin-based polymer [III]; and a powder obtained by agitating a flake-shaped brominated alkylphenol formaldehyde resin (produced by Taoka Chemical Co., Ltd., trade name: Tackirol 250-III) in a Henschel mixer for 10 seconds as the crosslinking agent [IV] were prepared.

Then, 83% by mass of polyamide [I-1], 12% by mass of the copolymer rubber [II], 3% by mass of olefin-based polymer [III-1], 2% by mass of the crosslinking agent [IV] and a small amount of a crosslinking aid (manufactured by Hakusuitech Co., Ltd., zinc oxide JIS#2) were pre-mixed, fed to a twin screw extruder (manufactured by Japan Steel Works, LTD., TEX-30), and melt kneaded at a cylinder temperature of 280°C and a screw rotation number of 300 rpm. A strand extruded from this twin screw extruder was cut to provide pellets of the polyamide-based thermoplastic elastomer composition.

The pellets thus obtained were used to perform the respective evaluation tests. The results are shown in Table 2.

Furthermore, extrusion molding of the pellets was performed using an extrusion molding machine (manufactured by Japan Steel Works, LTD., screw diameter: 30 mm) at a cylinder temperature of 250 to 340°C, to provide a monolayer tube having an outer diameter of 1/2 inches and a thickness of 1 mm. The monolayer tube thus obtained was used to perform the evaluation test of ethanol permeation properties. The results are shown in Table 2.

<Examples B2 to B10> (Examples B6 to B10 are comparative examples)

Respective pellets were produced in the same manner as in Example B1 except that the kinds of the polyamide [I] and the olefin-based polymer [III] used and the compounding ratio thereof were changed as shown in Table 2, and the evaluation tests were performed. The results are shown in Table 2.

Table 2

| | | Example | | | | | | | | | |
|---|---|---|---|---|---|---|---|---|---|---|---|
| | | B1 | B2 | B3 | B4 | B5 | B6 | B7 | B8 | B9 | B10 |
| Polyamide [I] | Kind | I-1 | I-2 | I-2 | I-3 | I-2 | I-5 | I-2 | I-2 | I-3 | I-2 |
| | Melting point(°C) | 276 | 264 | 264 | 265 | 264 | 295 | 264 | 264 | 265 | 264 |
| | Amount (mass%) | 83 | 68 | 47 | 68 | 68 | 68 | 85 | 70 | 70 | 68 |
| Copolymer rubber[II] | Amount (mass%) | 12 | 24 | 40 | 24 | 24 | 24 | 12 | 24 | 24 | 24 |
| Olefin polymer [III] | Kind | III-1 | III-1 | III-1 | III-1 | III-2 | III-1 | III-1 | III-1 | III-1 | III-3 |
| | Amount (mass%) | 3 | 6 | 10 | 6 | 6 | 6 | 3 | 6 | 6 | 6 |
| Crosslinking agent [IV] | Amount (mass%) | 2 | 2 | 3 | 2 | 2 | 2 | - | - | - | 2 |
| Proportion of rubber component in composition (mass%) | | 15 | 30 | 50 | 30 | 30 | 30 | 15 | 30 | 30 | 30 |
| Physical properties | Flexural modulus (MPa) | 1950 | 1130 | 400 | 760 | 1100 | Not molded | 2200 | 1540 | 1150 | 1310 |
| | Tensile elongation (%) | 40.3 | 44.5 | 49.8 | 48.7 | 40.8 | | 20.3 | 18.5 | 19.1 | 16.2 |
| | Tensile strength (MPa) | 71 | 44 | 20 | 35 | 42 | | 76 | 56 | 42 | 25 |
| CE10 fuel permeability coefficient | (g • mm/$m^2$ -24hr) | 4.4 | 7.1 | 8.8 | 8.1 | 7.7 | Not measured | 7.3 | 39.1 | 80.1 | 35.1 |
| M15 mass change rate | (%) | 3 | 13.5 | 15.9 | 14.7 | 14 | Not measured | 24.4 | 38.4 | 42.3 | 40.2 |
| Ethanol permeation properties of monolayer tube (g/24hr) | | 2.2 | 2.9 | 4.2 | 4.4 | 3.3 | Not measured | 4.1 | 13.2 | 19 | 16.1 |
| Examples B6 to B10 are comparative examples | | | | | | | | | | | |

Evaluation of physical properties in each Examples was performed according to the following methods.

[Flexural Modulus]

Injectionn molding of th pellets was performed using an injection molding machine (manufactured by Sodick Plustech Co., Ltd., apparatus name: Tuparl TR40S3A) under conditions of a cylinder temperature of [fusion end temperature (T) + 10]°C and a mold temperature of 40°C to produce a test piece having a thickness of 3 mm, and this test piece was further left to stand at a temperature of 23°C under a nitrogen atmosphere for 24 hours. Next, this test piece was subjected to a bending test using a bending test machine (manufactured by NTESCO, apparatus name: AB5) under an atmosphere of a temperature of 23°C and a relative humidity of 50% under conditions of a span of 51 mm and a bending speed of 1.4 mm/min to measure the flexural modulus (MPa).

[Tensile elongation, tensile strength]

The same apparatus and molding conditions as in the test piece of the flexural modulus measurement were used to produce a test piece having a thickness of 3 mm according to ASTM-1 (dumbbell piece), and left to stand under the same conditions for 24 hours. Next, this test piece was subjected to the tensile test under the same temperature and humidity conditions to measure the tensile elongation (%) and the tensile strength (MPa).

[CE10 fuel permeability coefficient]

Compression molding of the pellets was performed using a heat press machine under conditions of a press temperature of [fusion end temperature (T) + 5]°C and a press pressure of 3 MPa to produce a sheet having a thickness of 0.5 mm, and a disc-shaped test piece having a diameter of 100 mm was cut out from this sheet. This disc-shaped test piece was set at the opening of a SUS container (volume: 20 mL, opening area: $1.26 \times 10^{-3} m^2$) including 18 mL of CE10 (toluene/isooctane/ethanol = 45/45/10% by volume) as a simulated fuel, and sealed to provide a test body. This test body was placed in a constant-temperature apparatus (60°C), the mass of the test body was measured, and once the mass reduction per unit time was constant, the fuel permeability coefficient ($g \cdot mm/m^2 \cdot day$) was calculated by the following equation:

$$\text{Fuel permeability coefficient} = \{[\text{Mass reduced (g)}] \times [\text{Sheet thickness (mm)}]\}/\{\text{Opening area: } 1.26 \times 10^{-3}\ (m^2)] \times [\text{Measurement interval (day)}]\}.$$

[M15 mass change rate]

The same apparatus and molding conditions as in the test piece of the flexural modulus measurement were used to produce a test piece having a length of 35 mm, a width of 25 mm and a thickness of 0.5 mm, and this test piece was further left to stand under a nitrogen atmosphere at a temperature of 150°C for 1 hour. This test piece was immersed in 0.5 L of M15 (toluene/isooctane/methanol = 42.5/42.5/15% by volume) as a simulant fuel placed in an autoclave, and the lid of the autoclave was closed. The autoclave was warmed in a water tank at 60°C, the test piece was periodically removed from the autoclave and the mass change of the test piece was measured, and immersion was continued until the mass change of the test piece was not observed (saturated state). The M15 mass change rate (%) was calculated from the difference between the mass ($W_0$) before immersion and the mass ($W_1$) in the saturated state by the following equation:

$$\text{M15 mass change rate} = (W_1 - W_0)/W_0 \times 100.$$

[Ethanol permeation properties of monolayer tube]

The monolayer tube obtained in each of Examples and Comparative Examples was cut to a length of 30 cm, one end thereof was tightly stoppered, ethanol was placed thereinto, the other end was tightly stoppered, and the total weight was measured. Next, this tube was placed in an oven at 60°C, the weight change (g) after 24 hours was measured, and the ethanol permeation properties (g/24 hr) were evaluated based on the measurement value.

<Evaluation>

It is found from the values of respective physical properties [flexural modulus, tensile elongation and tensile strength] that sufficient flexibility is achieved in each of Examples B1 to B5 regardless of use of no plasticizer. It is also found that the M15 mass change rate is low and therefore there is little swelling due to the fuel, and the CE10 fuel permeability coefficient and the ethanol permeation properties are low and therefore the fuel and the solvent are hardly permeated. Accordingly, it can be said that a resin composition having comprehensively excellent characteristics as a resin composition for an industrial tube was obtained in each of Examples B1 to B5.

On the other hand, in Example B6, the melting point (Tm) of polyamide [1-5] used was too high to perform extrusion molding, and respective physical properties could not be measured. In each of Examples B7 to B9, no crosslinking agent [IV] was used, and therefore the hardness was high, the M15 mass change rate was high, and the CE10 fuel permeability coefficient and the ethanol permeation properties were also high as compared with corresponding Example (namely, Example where the same proportion of the rubber component and the same resin were adopted). In Example B10, unmodified olefin-based polymer [III-3] was used, and therefore the tensile elongation was reduced, the M15 mass change rate was high, and the CE10 fuel permeability coefficient and the ethanol permeation properties were also high as compared with Examples B2, B4 and B5 where the same proportion of the rubber component was adopted.

<Example C>

In the same manner as in Example A1, polyamide [1-2] as the polyamide [I]; an ethylene-propylene-5-ethylidene-2-norbornene copolymer rubber ([$\eta$] = 2.4 dl/g, ethylene content: 65% by mass, diene content: 4.6% by mass) as the copolymer rubber [II]; the modified polyolefin (maleic anhydride modified ethylene-1-butene copolymer, amount of maleic anhydride grafting modification (content rate of functional group structural unit): 0.97% by mass, intrinsic viscosity [$\eta$] measured in decalin solution at 135°C: 1.98 dl/g) synthesized in Example A1 as the olefin-based polymer [III]; and a powder obtained by agitating a flake-shaped brominated alkylphenol formaldehyde resin (produced by Taoka Chemical Co., Ltd., trade name: Tackirol 250-III) in a Henschel mixer for 10 seconds as the crosslinking agent [IV] were prepared.

Then, 47% by mass of the polyamide [1-2], 40% by mass of the copolymer rubber [II], 10% by mass of the olefin-based polymer [III], 3% by mass of the crosslinking agent [IV] and a small amount of a crosslinking aid (manufactured by Hakusuitech Co., Ltd., zinc oxide JIS#2) were pre-mixed, fed to a twin screw extruder (manufactured by Japan Steel Works, LTD., TEX-30), and melt kneaded under the following melting conditions (referred to as reference melting conditions). A strand extruded from this twin screw extruder was cut to provide pellets P1 of a polyamide-based thermoplastic elastomer composition.

(Reference melting conditions)

1) Cylinder temperature of extruder = 280°C
2) Barrel inner diameter D of extruder = 32 mm
3) Screw rotation number N = 350 rpm

Respective pellets P2 to P5 were obtained in the same manner as in pellets P1 except that the screw rotation number N or the cylinder temperature of the extruder was changed as shown in Table 3.

Respective characteristics of thus pelletized elastomer composition were evaluated. The results are shown in Table 3.

[Observation of film by transmission electron microscope (TEM)]

The cross section of each pellet was polished by a microtome to trim an ultrathin strip of the film cross section, and thereafter exposed to vapor of ruthenium tetroxide for a certain time to selectively stain one section. Each pellet was observed using a transmission electron microscope (TEM, H-7650 manufactured by Hitachi High-Technologies Corporation) at a magnification of 3000. The average particle size, the area image-analyzed, and the total area, where the particle size was 5 $\mu$m or more, of a particle (particle group (A)) stained were determined from the TEM image, and the ratio of the particle having a particle size of 5 $\mu$m or more in the area analyzed was calculated.

[Tensile creep test]

The test piece of the flexural modulus measurement was produced by the same method from each pellet, and

punched to provide a JIS K7162-1BA type dumbbell specimen. Next, this dumbbell specimen was subjected to a creep test at a tensile stress of 15 MPa for 30 minutes under an atmosphere of a temperature of 23°C and a relative humidity of 50%. The creep properties were rated according to the following evaluation criteria.

A: Creep strain was less than 10%

B: Creep strain was 10% or more

C: Breaking due to creep strain

[Evaluation method of die drool]

Extrusion molding of respective pellets was performed at a cylinder temperature of 290°C using an extrusion molding machine (manufactured by Thermoplastics Kogyo K.K., screw diameter: 20 mm) equipped with a rectangular die having a width of 15 mm and a thickness of 1 mm, to provide a sheet-shaped molded article. Here, the occurrence of die drool near the die was confirmed.

Herein, the occurrence of die drool was rated according to the following evaluation criteria.

A: Occurrence of die drool having a size of a length of one side of 1 mm or more was not confirmed even after lapse of 30 minutes after the initiation of sheet extrusion.

B: Occurrence of die drool having a size of a length of one side of 1 mm or more was confirmed with ultralow volume after lapse of 5 minutes to 30 minutes after the initiation of sheet extrusion.

C: Occurrence of die drool having a size of a length of one side of 1 mm or more was confirmed with a few amount after lapse of 5 minutes to 30 minutes after the initiation of sheet extrusion.

D: Die drool having a size of 1mm or more occurred over the entire periphery of die hole within 5 minutes after the initiation of sheet extrusion.

[Pressure proof test of tube]

A tube obtained by cutting a tubular molded body having an outer diameter of 8 mm and a wall thickness of 1 mm to a length of 50 cm was conditioned in a water tank adjusted at 40°C. Thereafter, one end of the tube was tightly stoppered and the other end was connected to a pressure apparatus for performing air degassing. Thereafter, application of pressure was performed at a test stress of an initial pressure of 0.5 MPa for 3 minutes, and when no breaking was observed, a test where the pressure was gradually increased in increments of 0.5 MPa and held at that pressure for 3 minutes was continuously performed and the internal pressure P ($kg/cm^2$) was calculated from the test stress in breaking of the tube. The pressure proof was rated according to the following evaluation criteria.

A: Internal pressure P at break was 10 $kg/cm^2$ or more

B: Internal pressure P at break was 5 $kg/cm^2$ or more and less than 10 $kg/cm^2$

C: Internal pressure P at break was less than 5 $kg/cm^2$

Table 3

| | | | Pellet | | | | |
|---|---|---|---|---|---|---|---|
| | | | P1 | P2 | P3 | P4 | P5 |
| Screw rotation speed | | rpm | 350 | 300 | 200 | 100 | 350 |
| Cylinder temperature | | °C | 280 | 280 | 280 | 280 | 310 |
| Average particle diameter particle group (A) | | μm | 1.54 | 2.04 | 2.62 | 4.52 | 1.84 |
| Area image-analyzed | | $\mu m^2$ | 4072 | 4072 | 3675 | 4022 | 4048 |
| Total area, where the particle size was 5 mm or more | | 2 $\mu m^2$ | 0 | 70.9 | 279 | 2360 | 973 |
| Ratio of the particle having a particle size of 5 μm or more in the area analyzed | | % | 0.00 | 1.74 | 7.59 | 58.68 | 24.03 |
| Evaluation | Tensile creep | | A | A | B | B | A |
| | Die drool | | A | A | B | C | C |
| | Tube pressure proof | | A | A | B | B | A |

<Evaluation>

Each of pellets P1 to P3, in which the proportion of the cumulative cross sectional area of the area of the particle of the dispersion component (particle group (A)) having a particle size of 5 μm or more to the entire cross sectional area analyzed was 10% or less, caused slight die drool to occur, and was good. On the other hand, each of pellets P4 and P5, in which the proportion was more than 10%, caused much die drool to occur. When the proportion of the cumulative cross sectional area was 5% or less, furthermore 2.5% or less, excellent tensile creep properties and tube pressure proof were achieved, and in particular, pellet P1, in which the proportion of the cumulative cross sectional area was 0%, namely, a particle group of 5 μm or more was not present, was excellent in all of the tensile creep properties, suppression of die drool and tube pressure proof.

<Example D: Polyamide-based thermoplastic elastomer composition [Z]>

<Production Example Y-1>

Polyamide [1-2] was prepared as the polyamide [I].

An ethylene/propylene/5-ethylidene-2-norbornene copolymer rubber ([η] = 2.4 dl/g, ethylene content: 65% by mass, diene content: 4.6% by mass) was prepared as the copolymer rubber [II].

A modified polyolefin (maleic anhydride modified ethylene-1-butene copolymer, amount of maleic anhydride grafting modification (content rate of functional group structural unit): 0.97% by mass, intrinsic viscosity [η] measured in decalin solution at 135°C: 1.98 dl/g), synthesized as follow, was prepared as the olefin-based polymer [III].

First, a glass flask sufficiently purged with nitrogen was charged with 0.63 mg of bis(1,3-dimethylcyclopentadienyl)zirconium chloride, and 1.57 ml of a solution of methylaluminoxane in toluene (Al; 0.13 mmol/l) and 2.43 ml of toluene were further added to thereby provide a catalyst solution. To a stainless autoclave having an inner volume of 2 L, sufficiently purged with nitrogen, 912 ml of hexane and 320 ml of 1-butene were introduced, and the temperature in the system was raised to 80°C. Subsequently, 0.9 mmol of triisobutylaluminum and 2.0 ml of the above catalyst solution (0.0005 mmol as Zr) were loaded under pressure by ethylene to initiate polymerization. Ethylene was continuously fed to thereby keep the total pressure at 8.0 kg/cm$^2$-G, and polymerization was performed at 80°C for 30 minutes. A small amount of ethanol was introduced into the system to stop the polymerization, and thereafter unreacted ethylene was purged from the system. The resulting solution was poured into a large excess of methanol to thereby allow a white solid to be precipitated. This white solid was collected by filtration, and dried under reduced pressure overnight to provide a white solid ethylene-1-butene copolymer. In this ethylene-1-butene copolymer, the density was 0.862 g/cm$^3$, the MFR (ASTM D1238 standard, 190°C, load: 2160 g) was 0.5 g/10 min, and the content rate of a 1-butene structural unit was 4% by mole. This ethylene-1-butene copolymer (100 parts by mass) was mixed with 1.0 part by mass of maleic anhydride and 0.04 parts by mass of peroxide (produced by NOF Corporation, trade name: Perhexyne 25B), and the resulting mixture was subjected to melt grafting modification in a single screw extruder set at 230°C, to thereby provide the maleic anhydride modified ethylene-1-butene copolymer.

A powder obtained by agitating a flake-shaped brominated alkylphenol formaldehyde resin (produced by Taoka Chemical Co., Ltd., trade name: Tackirol 250-III) by a Henschel mixer for 10 seconds was prepared as the crosslinking agent [IV].

Then, 40% by mass of polyamide [1-2], 45% by mass of the copolymer rubber [II], 12% by mass of the olefin-based polymer [III], 3% by mass of the crosslinking agent [IV] and a small amount of a crosslinking aid (manufactured by Hakusuitech Co., Ltd., zinc oxide JIS#2) were pre-mixed, and the resultant was fed to a twin screw extruder (manufactured by Japan Steel Works, LTD., TEX-30) and melt kneaded at a cylinder temperature of 280°C and a number of screw rotations of 300 rpm. A strand extruded from this twin screw extruder was cut to provide pellets of a polyamide-based resin composition [Y-1].

The pellets thus obtained were used to perform measurement of the flexural modulus. The results are shown in Table 4.

<Production Examples Y-2 to Y-7>

Respective pellets were produced in the same manner as in Production Example Y-1 except that the kinds of the polyamide [I] used and the compounding ratio of each component were changed as shown in Table 4, and measurement of the flexural modulus was performed. The results are shown in Table 4.

Table 4

| | | Production Example | | | | | | |
|---|---|---|---|---|---|---|---|---|
| | | Y-1 | Y-2 | Y-3 | Y-4 | Y-5 | Y-6 | Y-7 |
| Polyamide [I] | Kind | I-2 | I-2 | I-3 | I-5 | I-3 | I-2 | I-2 |
| | Melting point (°C) | 264 | 264 | 265 | 295 | 265 | 264 | 264 |
| | Amount (mass%) | 40 | 30 | 40 | 40 | 41 | 40 | 40 |
| Copolymer rubber [II] | Amount (mass%) | 45 | 55 | 45 | 45 | 46 | 45 | 45 |
| Olefin polymer [III] | Kind | III-1 | III-1 | III-1 | III-1 | III-1 | III-2 | III-3 |
| | Amount (mass%) | 12 | 12 | 12 | 12 | 13 | 12 | 12 |
| Crosslinking agent [IV] | Amount (mass%) | 3 | 3 | 3 | 3 | - | 3 | 3 |
| Proportion of rubber component in composition (mass%) | | 57 | 67 | 57 | 57 | 59 | 57 | 57 |
| Physical property | Flexural modulus (MPa) | 220 | 110 | 160 | 350 | 410 | 240 | 390 |

[Flexural Modulus]

Injection molding of the pellets was performed using an injection molding machine (manufactured by Sodick Plustech Co., Ltd., apparatus name: Tuparl TR40S3A) under conditions of a cylinder temperature of [fusion end temperature (T) + 10]°C and a mold temperature of 40°C to produce a test piece having a thickness of 3 mm, and this test piece was further left to stand at a temperature of 23°C under a nitrogen atmosphere for 24 hours. Next, this test piece was subjected to a bending test using a bending test machine (manufactured by NTESCO, apparatus name: AB5) under an atmosphere of a temperature of 23°C and a relative humidity of 50% under conditions of a span of 51 mm and a bending speed of 1.4 mm/min to measure the flexural modulus (MPa).

[Hardness (JIS-D)]

Measurement was made on a durometer D scale according to JIS K7215.

[Tensile break strength, elongation between bench marks at tensile break]

Measurement was performed (n = 3) according to the method described in JIS K6251, and the average value with respect to each of the strength (MPa) and the elongation (%) at break of the test piece was adopted. As the test piece, a test piece having a shape of No. 2 (1/3) and a thickness of about 2 mm was used. The test was performed at 23°C and a testing speed of 500 mm/min. The test piece was in principle conditioned before the test at a temperature of 23°C ± 2°C and a relative humidity of 50 ± 5% for 48 hours or more, and then used.

[Elasticity retention rate at high temperature (%)]

Measurement of the flexural modulus was performed at 120°C, and the elasticity retention rate at a high temperature was calculated by the following equation 1.

$$\text{(Equation 1): Flexural Modulus (120°C)/Flexural Modulus (23°C)} \times 100$$

[Oil resistance (weight change rate/%)]

The molded body of the composition was immersed in IRM903 oil kept at 140°C for 72 hours and the weight change rate (% by weight) was determined according to JIS K6258.

[Water absorption rate (%)]

Measurement was made under conditions of 23°C and 24 hours according to ASTM D570.

[Blow moldability]

Evaluation of the draw down properties in blow molding was performed as follows. A cylindrical (pipe-shaped) parison was extruded using a large-scaled blow molding machine (manufactured by Bekum, direct blow molding machine) with a die of φ70 mm and a mandrel of φ60 mm in a continuous manner without accumulator. The shape-imparting characteristics of the parison (solidification, elongation state) and the draw down state were rated according to the following criteria. Herein, molding conditions were as follows: the cylinder temperature was (fusion end temperature (T) + 10)°C and the mold temperature was 40°C. Air spray was performed for a spraying time of 10 seconds immediately after mold clamping.

"A"; The shape-imparting characteristics of the parison was stable and molded article was obtained without causing draw down.

"B"; Shaping of parison was possible, but draw down was significantly caused and remarkable unevenness in thickness was observed in molded article.

"C"; Shaping of parison was not stable and draw down was caused, and also remarkable molding failure (boring, breaking) was observed.

<Examples D1 to D13> (Examples D8 to D13 are comparative examples)

The polyamide [Z1] and polyamide-based resin composition [Z2] shown in Table 5, and 1.0 part by mass of a stabilizer (produced by Sumitomo Chemical Co., Ltd., Sumilizer #GA80) and 0.7 parts by mass of a crystal nucleating agent (produced by Matsumura Sangyo Co., Ltd., #ET-5) as other additives were fed to a 35φ twin screw extruder (manufactured by Toshiba Machine Co., Ltd.), and melt kneaded at a cylinder temperature of 280°C and a screw rotation number of 300 rpm. A strand extruded from this twin screw extruder was cut to provide pellets of a polyamide-based thermoplastic elastomer composition [Z]. The pellets were used to perform the respective evaluation tests. The results are shown in Table 5.

Table 5

| | | Example | | | | | | | | | | | | |
|---|---|---|---|---|---|---|---|---|---|---|---|---|---|---|
| | | D1 | D2 | D3 | D4 | D5 | D6 | D7 | D8 | D9 | D10 | D11 | D12 | D13 |
| Polyamide [Z1] | Kind | Z1-1 | Z1-2 | Z1-2 | Z1-2 | Z1-3 | Z1-3 | Z1-2 | Z1-2 | Z1-2 | Z1-2 | Z1-2 | Polyester elastome | Z1-2 |
| | Melting point(°C) | 225 | 265 | 265 | 265 | 243 | 243 | 265 | 265 | 265 | 265 | 265 | 202 | 265 |
| | Amount (mass%) | 20 | 20 | 20 | 20 | 20 | 30 | 20 | 20 | 20 | 5 | 40 | 100 | 20 |
| Polyamide-based resin composition [Z2] | Kind | Y-2 | Y-1 | Y-2 | Y-3 | Y-2 | Y-2 | Y-6 | Y-4 | Y-5 | Y-2 | Y-2 | - | Y-7 |
| | Melting point(°C) | 264 | 264 | 264 | 265 | 264 | 264 | 264 | 295 | 265 | 264 | 264 | - | 264 |
| | Amount (mass%) | 80 | 80 | 80 | 80 | 80 | 70 | 80 | 80 | 80 | 95 | 60 | - | 80 |
| Physical properties | Hardness (JIS-D) | 39 | 42 | 38 | 38 | 40 | 35 | 42 | 41 | 44 | 25 | 70 | 36 | 49 |
| | Tensile breaking strength (MPa) | 21.2 | 25.1 | 32.1 | 22.8 | 29.2 | 33.5 | 24 | 22.5 | 14.5 | 14.4 | 37.2 | 35.2 | 21.1 |
| | Elongation between bench marks at tensile break (%) | 49 | 50 | 58 | 72 | 51 | 66 | 48 | 39 | 32 | 41 | 16 | 80 | 30 |
| | Flexural modulus (MPa) | 240 | 360 | 220 | 240 | 340 | 230 | 380 | 400 | 610 | 150 | 1050 | 220 | 500 |
| | Elasticity retention rate at high temperature (%) | 54 | 60 | 66 | 62 | 56 | 64 | 60 | 46 | 42 | 36 | 34 | 32 | 32 |
| | Oil resistance (weight change rate/%) | 10.5 | 8.4 | 11.1 | 12.5 | 9.2 | 7.6 | 8.1 | 10.3 | 42.7 | 34.2 | 7.1 | 22.0 | 72.2 |
| | Water absorption rate (%) | 0.6 | 0.6 | 0.5 | 0.3 | 0.6 | 0.8 | 0.6 | 0.6 | 0.5 | 0.5 | 1.1 | 0.8 | 1.1 |
| Blow moldability | | A | A | A | A | A | A | A | C | C | B | B | A | C |

Examples D8 to D13 are comparative examples

Polyamides [Z1-1] to [Z1-3] and the polyester elastomer in Table 5 specifically represent the following commercially available products.

[Z1-1]: Polycaproamide (nylon 6) (produced by Toray Industries, Inc., trade name: Amilan "CM1046", melting point = 225°C, molten heat capacity ($\Delta H$) = 64 mJ/mg)

[Z1-2]: Polyhexamethylene adipamide (nylon 66) (produced by Toray Industries, Inc., trade name: Amilan "CM3001-N", melting point = 265°C, molten heat capacity ($\Delta H$) = 66 mJ/mg)

[Z1-3]: Copolymer (produced by Mitsubishi Engineering-Plastics Corporation, trade name: polyamide MXD6 Reny "#6002", melting point = 243°C, molten heat capacity ($\Delta H$) = 52 mJ/mg) of salt of metaxylylenediamine and adipic acid

"Polyester elastomer": Thermoplastic polyether ester elastomer (TPEE) (produced by Du Pont-Toray Co., Ltd., trade name: Hytrel "HTR-4275", melting point = 202°C, molten heat capacity ($\Delta H$) = 36.2 mJ/mg)

<Evaluation>

In each of Examples D1 to D7, excellent results were obtained with respect to all of the evaluation items. Accordingly, it can be said that a composition having comprehensively excellent characteristics as a material for a molded article (for example, automobile constant velocity joint boot) required to have such physical properties was obtained.

On the other hand, in Example D8, polyamide-based resin composition [Y-4] including polyamide [I-5] having a too high melting point was used, and therefore the elasticity retention rate at a high temperature was poor and blow molding could not be performed. In Example D9, no crosslinking agent [IV] was used and polyamide-based resin composition [Y-5] not dynamically crosslinked was used, and therefore the elasticity retention rate at a high temperature and the oil resistance were poor, and blow molding could not be performed. In Example D10, the amount of the polyamide [Z1] was too small, and therefore the elasticity retention rate at a high temperature, the oil resistance and the blow moldability were poor. In Example D11, the amount of the polyamide [Z1] was too large, and therefore the elasticity retention rate at a high temperature and the blow moldability were poor. Example D12 was an example using a commercially available polyester elastomer singly, and the elasticity retention rate at a high temperature and the oil resistance were poor. In Example D13, polyamide-based resin composition [Y-7] including unmodified olefin-based polymer [III-3] was used, and therefore the oil resistance was poor, the water absorption rate was also high, the elasticity retention rate at a high temperature was poor and blow molding could not be performed.

Industrial Applicability

While the polyamide-based thermoplastic elastomer composition [Y] of the present invention is described with being classified to two elastomer compositions [Y1] and [Y2] depending on the compositional ranges of the components [I], [II], [III] and [IV], overlapping of [Y1] and [Y2] is not problematic. Accordingly, the elastomer composition [Y1] can be used as the polyamide-based resin composition [Z2] of the polyamide-based thermoplastic elastomer composition [Z].

## Claims

1. A polyamide-based thermoplastic elastomer composition [Y], in which a rubber composition [X] and a phenol resin-based crosslinking agent [IV] are dynamically crosslinked,
wherein the rubber composition [X] comprises:

    a polyamide [I] comprising 30 to 100 % by mole of a structural unit derived from terephthalic acid in total structural units of dicarboxylic acids and a melting point (Tm) determined by differential scanning calorimetry (DSC) of the polyamide being 220 to 290°C,
    an ethylene-α-olefin-unconjugated polyene copolymer rubber [II] comprising structural units derived from ethylene [a], an α-olefin [b] having 3 to 20 carbon atoms, and an unconjugated polyene [c] having at least one carbon-carbon double bond in one molecule, respectively, and
    an olefin-based polymer [III] comprising 0.3 to 5.0% by mass of a functional group structural unit in a molecule.

2. The polyamide-based thermoplastic elastomer composition [Y] according to claim 1, wherein the polyamide [I] further comprises a structural unit derived from isophthalic acid as a component of the dicarboxylic acid, and a structural unit derived from an aliphatic diamine having 4 to 15 carbon atoms as a diamine component.

3. The polyamide-based thermoplastic elastomer composition [Y] according to claim 2, wherein a molar ratio of the structural unit derived from terephthalic acid/the structural unit derived from isophthalic acid in the polyamide [I] is 65/35 to 50/50.

4. The polyamide-based thermoplastic elastomer composition [Y] according to any one of claims 1 to 3, wherein 40 to 100% by mole of a total of a diamine component comprised in the polyamide [I] is a structural unit derived from 1,6-hexanediamine.

5. The polyamide-based thermoplastic elastomer composition [Y] according to any one of claims 1 to 4, wherein the functional group structural unit in the olefin-based polymer [III] comprises a functional group selected from the group consisting of a carboxylic acid group, an ester group, an ether group, an aldehyde group and a ketone group.

6. The polyamide-based thermoplastic elastomer composition [Y] according to any one of claims 1 to 4, wherein the functional group structural unit in the olefin-based polymer [III] is a maleic anhydride structural unit.

**7.** The polyamide-based thermoplastic elastomer composition [Y] according to any one of claims 1 to 6, comprising:

the polyamide [I] as a matrix component; and
a dispersion component dispersed in the matrix component, the dispersion component being a particle comprising the ethylene-α-olefin-unconjugated polyene copolymer rubber [II] and the olefin-based polymer [III], and the dispersion component being satisfied the following (1):

(1) a cross sectional area of the particle, in which a particle size is 5 μm or more, of the dispersion component is measured, and a proportion of a cumulative cross sectional area of the area of the particle to an entire cross sectional area analyzed is 10% or less.

**8.** The polyamide-based thermoplastic elastomer composition [Y1] according to any one of claims 1 to 6, in which a rubber composition [X] comprising:

10 to 60% by mass of the polyamide [I];
30 to 86% by mass of the ethylene-α-olefin-unconjugated polyene copolymer rubber [II]; and
3 to 30% by mass of the olefin-based polymer [III], and

1 to 10% by mass of the phenol resin-based crosslinking agent [IV] are dynamically crosslinked, provided that a total amount of [I], [II], [III] and [IV] is 100% by mass.

**9.** The polyamide-based thermoplastic elastomer composition [Y1] according to claim 8, wherein a mass ratio ([II]/[III]) of the ethylene-α-olefin-unconjugated polyene copolymer rubber [II] to the olefin-based polymer [III] in the rubber composition [X] is 95/5 to 60/40.

**10.** The polyamide-based thermoplastic elastomer composition [Y1] according to claim 8 or 9, wherein 10% or more of an end group of a molecular chain of the polyamide [I] is terminated by an end-terminating agent, and an amount of a terminal amino group of the molecular chain is 0.1 to 100 mmol/kg.

**11.** A molded article obtained from the polyamide-based thermoplastic elastomer composition [Y1] according to any one of claims 8 to 10.

**12.** The polyamide-based thermoplastic elastomer composition [Y2] according to any one of claims 1 to 6, in which a rubber composition [X] comprising:

30 to 87.7% by mass of the polyamide [I];
10 to 45% by mass of the ethylene-α-olefin-unconjugated polyene copolymer rubber [II]; and
2 to 20% by mass of the olefin-based polymer [III], and

0.3 to 5.0% by mass of the phenol resin-based crosslinking agent [IV] are dynamically crosslinked, provided that a total amount of [I], [II], [III] and [IV] is 100% by mass.

**13.** An industrial tube having at least a layer comprising the polyamide-based thermoplastic elastomer composition [Y2] according to claim 12.

**14.** The industrial tube according to claim 13, which is a tube for automobile piping, a pneumatic tube, a hydraulic tube, a paint spray tube or a medical tube.

**15.** The industrial tube according to claim 13 or 14, having an outer diameter of 2 mm to 50 mm and a thickness of 0.2 mm to 10 mm.

**16.** The industrial tube according to any one of claims 13 to 15, further comprising a layer comprising at least one resin selected from the group consisting of a fluororesin, a high density polyethylene resin, a polybutylene naphthalate resin (PBN), an aliphatic polyamide resin, an aromatic polyamide resin, a metaxylene group-containing polyamide resin, an ethylene-vinyl acetate copolymer saponified product (EVOH) and a polyphenylene sulfide resin (PPS).

**17.** A polyamide-based thermoplastic elastomer composition [Z] comprising 10 to 35% by mass of a polyamide [Z1] having a melting point (Tm) determined by differential scanning calorimetry (DSC) of 210 to 270°C and a molten

heat capacity (ΔH) determined by DSC of 45 to 80 mJ/mg, and 65 to 90% by mass of a polyamide-based resin composition [Z2], provided that a total amount of [Z1] and [Z2] is 100% by mass, wherein

the polyamide-based resin composition [Z2] is the polyamide-based thermoplastic elastomer composition [Y2] according to claim 12.

**18.** A molded article obtained from the polyamide-based thermoplastic elastomer composition [Z] according to claim 17 by injection molding or blow molding.

**19.** An automobile constant velocity joint boot comprising the polyamide-based thermoplastic elastomer composition [Z] according to claim 17.

**Patentansprüche**

**1.** Thermoplastische Polyamid-basierte Elastomerzusammensetzung [Y], in der eine Kautschukzusammensetzung [X] und ein Phenolharz-basiertes Vernetzungsmittel [IV] dynamisch vernetzt sind,
wobei die Kautschukzusammensetzung [X] umfasst:

ein Polyamid [I], umfassend 30 bis 100 Mol-% einer von Terephthalsäure abgeleiteten Struktureinheit in den gesamten Struktureinheiten der Dicarbonsäuren und einen Schmelzpunkt (Tm), bestimmt durch dynamische Differenzkalorimetrie (DSC) des Polyamids, von 220 bis 290°C,
einen Ethylen-α-Olefin-nichtkonjugiertes Polyen-Copolymerkautschuk [II], umfassend Struktureinheiten, abgeleitet von Ethylen [a], einem α-Olefin [b] mit 3 bis 20 Kohlenstoffatomen bzw. einem nichtkonjugierten Polyen [c] mit mindestens einer C-C-Doppelbindung in einem Molekül, und
ein Olefin-basiertes Polymer [III], umfassend 0,3 bis 5,0 Massen-% einer Struktureinheit mit funktioneller Gruppe in einem Molekül.

**2.** Thermoplastische Polyamid-basierte Elastomerzusammensetzung [Y] gemäß Anspruch 1, wobei das Polyamid [I] zusätzlich eine von Isophthalsäure abgeleitete Struktureinheit als eine Dicarbonsäurekomponente umfasst und eine von einem aliphatischen Diamin mit 4 bis 15 Kohlenstoffatomen abgeleitete Struktureinheit als eine Diaminkomponente umfasst.

**3.** Thermoplastische Polyamid-basierte Elastomerzusammensetzung [Y] gemäß Anspruch 2, wobei in dem Polyamid [I] das Molverhältnis der von Terephthalsäure abgeleiteten Struktureinheit/der von Isophthalsäure abgeleiteten Struktureinheit 65/35 bis 50/50 beträgt.

**4.** Thermoplastische Polyamid-basierte Elastomerzusammensetzung [Y] gemäß einem der Ansprüche 1 bis 3, wobei 40 bis 100 Mol-% der gesamten Diaminkomponente, die in dem Polyamid [I] enthalten ist, eine von 1,6-Hexandiamin abgeleitete Struktureinheit ist.

**5.** Thermoplastische Polyamid-basierte Elastomerzusammensetzung [Y] gemäß einem der Ansprüche 1 bis 4, wobei die Struktureinheit mit funktioneller Gruppe in dem Olefin-basierten Polymer [III] eine funktionelle Gruppe umfasst, die aus der Gruppe ausgewählt ist, die aus einer Carbonsäuregruppe, einer Estergruppe, einer Ethergruppe, einer Aldehydgruppe und einer Ketogruppe besteht.

**6.** Thermoplastische Polyamid-basierte Elastomerzusammensetzung [Y] gemäß einem der Ansprüche 1 bis 4, wobei die Struktureinheit mit funktioneller Gruppe in dem Olefin-basierten Polymer [III] eine Maleinsäureanhydrid-Struktureinheit ist.

**7.** Thermoplastische Polyamid-basierte Elastomerzusammensetzung [Y] gemäß einem der Ansprüche 1 bis 6, umfassend:

das Polyamid [I] als Matrixkomponente; und
eine Dispersionskomponente, die in der Matrixkomponente dispergiert ist, wobei die Dispersionskomponente ein Partikel ist, der den Ethylen-α-Olefin-nichtkonjugiertes Polyen-Copolymerkautschuk [II] und das Olefin-basierte Polymer [III] umfasst, und wobei die Dispersionskomponente das folgenden (1) erfüllt:

(1) eine Querschnittsfläche des Partikels der Dispersionskomponente, bei dem eine Partikelgröße 5 μm oder mehr beträgt, wird gemessen und ein Anteil einer kumulativen Querschnittsfläche der Partikelfläche zur gesamten analysierten Querschnittsfläche beträgt 10% oder weniger.

**8.** Thermoplastische Polyamid-basierte Elastomerzusammensetzung [Y1] gemäß einem der Ansprüche 1 bis 6, worin eine Kautschukzusammensetzung [X], umfassend:

10 bis 60 Massen-% Polyamid [I];
30 bis 86 Massen-% Ethylen-α-Olefinnichtkonjugiertes Polyen-Copolymerkautschuk [II]; und
3 bis 30 Massen-% Olefin-basiertes Polymer [III], und

1 bis 10 Massen-% Phenolharz-basiertes Vernetzungsmittel [IV] dynamisch vernetzt sind, vorausgesetzt, dass eine Gesamtmenge von [I], [II], [III] und [IV] 100 Massen-% beträgt.

**9.** Thermoplastische Polyamid-basierte Elastomerzusammensetzung [Y1] gemäß Anspruch 8, wobei in der Kautschukzusammensetzung [X] ein Massenverhältnis ([II]/[III]) von dem Ethylen-α-Olefin-nichtkonjugiertes Polyen-Copolymerkautschuk [II] zu dem Olefin-basierten Polymer [III] 95/5 bis 60/40 beträgt.

**10.** Thermoplastische Polyamid-basierte Elastomerzusammensetzung [Y1] gemäß Anspruch 8 oder 9, wobei 10% oder mehr einer Endgruppe einer Molekülkette des Polyamids [I] mittels eines Endenabbruchmittels abgeschlossen sind und eine Menge einer terminalen Aminogruppe der Molekülkette 0,1 bis 100 mmol/kg beträgt.

**11.** Formgegenstand, erhalten aus der thermoplastischen Polyamid-basierten Elastomerzusammensetzung [Y1] gemäß einem der Ansprüche 8 bis 10.

**12.** Thermoplastische Polyamid-basierte Elastomerzusammensetzung [Y2] gemäß einem der Ansprüche 1 bis 6, worin eine Kautschukzusammensetzung [X], umfassend:

30 bis 87,7 Massen-% Polyamid [I];
10 bis 45 Massen-% Ethylen-α-Olefin-nichtkonjugiertes Polyen-Copolymerkautschuk [II]; und
2 bis 20 Massen-% Olefin-basiertes Polymer [III], und

0,3 bis 5,0 Massen-% Phenolharz-basiertes Vernetzungsmittel [IV] dynamisch vernetzt sind, vorausgesetzt, dass eine Gesamtmenge von [I], [II], [III] und [IV] 100 Massen-% beträgt.

**13.** Industrieleitung mit mindestens einer Schicht, umfassend die thermoplastische Polyamid-basierte Elastomerzusammensetzung [Y2] gemäß Anspruch 12.

**14.** Industrieleitung gemäß Anspruch 13, die eine Leitung für Automobile, ein Pneumatikschlauch, ein Hydraulikschlauch, ein Farbspritzschlauch oder ein medizinischer Schlauch ist.

**15.** Industrieleitung gemäß Anspruch 13 oder 14, die einen Außendurchmesser von 2 mm bis 50 mm und eine Dicke von 0,2 mm bis 10 mm aufweist.

**16.** Industrieleitung gemäß einem der Ansprüche 13 bis 15, zusätzlich umfassend eine Schicht, umfassend mindestens ein Harz, ausgewählt aus der Gruppe, bestehend aus einem Fluorharz, einem Polyethylenharz mit hoher Dichte, einem Polybutylennaphthalatharz (PBN), einem aliphatischen Polyamid, einem aromatischen Polyamidharz, einem Metaxylengruppe-haltigen Polyamidharz, einem EthylenVinylacetat-Copolymer-Verseifungsprodukt (EVOH) und einem Polyphenylensulfidharz (PPS).

**17.** Thermoplastische Polyamid-basierte Elastomerzusammensetzung [Z], umfassend 10 bis 35 Massen-% Polyamid [Z1] mit einem Schmelzpunkt (Tm), bestimmt durch dynamische Differenzkalorimetrie (DSC), von 210 bis 270°C und einer Schmelzwärmekapazität (ΔH), bestimmt durch DSC, von 45 bis 80 mJ/mg und 65 bis 90 Massen-% einer Polyamid-basierten Harzzusammensetzung [Z2], vorausgesetzt, dass eine Gesamtmenge von [Z1] und [Z2] 100 Massen-% beträgt, wobei
die Polyamid-basierte Harzzusammensetzung [Z2] die thermoplastische Polyamid-basierte Elastomerzusammensetzung [Y2] gemäß Anspruch 12 ist.

**18.** Formgegenstand, erhalten aus der thermoplastischen Polyamid-basierten Elastomerzusammensetzung [Z] gemäß Anspruch 17 durch Spritzgießen oder Blasformen.

**19.** Gleichlaufgelenkmanschette für Automobile, umfassend die thermoplastische Polyamid-basierte Elastomerzusammensetzung [Z] gemäß Anspruch 17.

**Revendications**

**1.** Composition d'élastomère thermoplastique à base de polyamide [Y], dans laquelle une composition de caoutchouc [X] et un agent de réticulation à base de résine de phénol [IV] sont réticulés de manière dynamique,
dans laquelle la composition de caoutchouc [X] comprend :

un polyamide [I] comprenant 30 à 100 % en mole d'un motif structurel dérivé d'un acide téréphtalique dans des motifs structurels totaux d'acides dicarboxyliques et un point de fusion (Tm) déterminé par calorimétrie à balayage différentiel (DSC) du polyamide étant de 220 à 290 °C,
un caoutchouc de copolymère d'éthylène-α-oléfine-polyène non conjugué [II] comprenant des motifs structurels dérivés de l'éthylène [a], d'une α-oléfine [b] ayant 3 à 20 atomes de carbone, et d'un polyène non conjugué [c] ayant au moins une liaison double carbone-carbone dans une molécule, respectivement, et
un polymère à base d'oléfine [III] comprenant 0,3 à 5,0 % en masse d'un motif structurel à groupe fonctionnel dans une molécule.

**2.** Composition d'élastomère thermoplastique à base de polyamide [Y] selon la revendication 1, dans laquelle le polyamide [I] comprend en outre un motif structurel dérivé d'un acide isophtalique en tant que composant de l'acide dicarboxylique, et un motif structurel dérivé d'une diamine aliphatique ayant 4 à 15 atomes de carbone en tant que composant diamine.

**3.** Composition d'élastomère thermoplastique à base de polyamide [Y] selon la revendication 2, dans laquelle un rapport molaire du motif structurel dérivé d'un acide téréphtalique/motif structurel dérivé d'un acide isophtalique dans le polyamide [I] est 65/35 à 50/50.

**4.** Composition d'élastomère thermoplastique à base de polyamide [Y] selon l'une quelconque des revendications 1 à 3, dans laquelle 40 à 100 % en mole d'un total d'un composant diamine compris dans le polyamide [I] est un motif structurel dérivé de la 1,6-hexanediamine.

**5.** Composition d'élastomère thermoplastique à base de polyamide [Y] selon l'une quelconque des revendications 1 à 4, dans laquelle le motif structurel à groupe fonctionnel dans le polymère à base d'oléfine [III] comprend un groupe fonctionnel sélectionné dans le groupe constitué d'un groupe acide carboxylique, d'un groupe ester, d'un groupe éther, d'un groupe aldéhyde et d'un groupe cétone.

**6.** Composition d'élastomère thermoplastique à base de polyamide [Y] selon l'une quelconque des revendications 1 à 4, dans laquelle le motif structurel à groupe fonctionnel dans le polymère à base d'oléfine [III] est un motif structurel anhydride maléique.

**7.** Composition d'élastomère thermoplastique à base de polyamide [Y] selon l'une quelconque des revendications 1 à 6, comprenant :

le polyamide [I] en tant que composant de matrice ; et
un composant de dispersion dispersé dans le composant de matrice, le composant de dispersion étant une particule comprenant le caoutchouc de copolymère d'éthylène-α-oléfine-polyène non conjugué [II] et le polymère à base d'oléfine [III], et le composant de dispersion satisfaisant le (1) suivant :

(1) une surface en coupe transversale de la particule, dans laquelle une taille particulaire est de 5 μm ou plus, du composant de dispersion est mesurée, et une proportion d'une surface en coupe transversale cumulée de la surface de la particule par rapport à une surface en coupe transversale entière analysée est de 10 % ou moins.

**8.** Composition d'élastomère thermoplastique à base de polyamide [Y1] selon l'une quelconque des revendications 1

à 6, dans laquelle
une composition de caoutchouc [X] comprenant :

10 à 60 % en masse du polyamide [I] ;
30 à 86 % en masse du caoutchouc de copolymère d'éthylène-$\alpha$-oléfine-polyène non conjugué [II] ; et
3 à 30 % en masse du polymère à base d'oléfine [III], et

1 à 10 % en masse de l'agent de réticulation à base de résine de phénol [IV] sont réticulés de manière dynamique, à condition qu'une quantité totale de [I], [II], [III] et [IV] soit de 100 % en masse.

**9.** Composition d'élastomère thermoplastique à base de polyamide [Y1] selon la revendication 8, dans laquelle un rapport en masse ([II]/[III]) du caoutchouc de copolymère d'éthylène-$\alpha$-oléfine-polyène non conjugué [II] sur le polymère à base d'oléfine [III] dans la composition de caoutchouc [X] est de 95/5 à 60/40.

**10.** Composition d'élastomère thermoplastique à base de polyamide [Y1] selon la revendication 8 ou 9, dans laquelle 10 % ou plus d'un groupe terminal d'une chaîne moléculaire du polyamide [I] sont terminés par un agent de terminaison d'extrémité, et une quantité d'un groupe amino terminal de la chaîne moléculaire est de 0,1 à 100 mmol/kg.

**11.** Article moulé obtenu à partir de la composition d'élastomère thermoplastique à base de polyamide [Y1] selon l'une quelconque des revendications 8 à 10.

**12.** Composition d'élastomère thermoplastique à base de polyamide [Y2] selon l'une quelconque des revendications 1 à 6, dans laquelle
une composition de caoutchouc [X] comprenant :

30 à 87,7 % en masse du polyamide [I] ;
10 à 45 % en masse du caoutchouc de copolymère d'éthylène-$\alpha$-oléfine-polyène non conjugué [II] ; et
2 à 20 % en masse du polymère à base d'oléfine [III], et
0,3 à 5,0 % en masse de l'agent de réticulation à base de résine de phénol [IV] sont réticulés de manière dynamique, à condition qu'une quantité totale de [I], [II], [III] et [IV] soit de 100 % en masse.

**13.** Tube industriel ayant au moins une couche comprenant la composition d'élastomère thermoplastique à base de polyamide [Y2] selon la revendication 12.

**14.** Tube industriel selon la revendication 13, qui est un tube pour tuyauterie d'automobile, un tube pneumatique, un tube hydraulique, un tube de pulvérisation de peinture ou un tube médical.

**15.** Tube industriel selon la revendication 13 ou 14, ayant un diamètre externe de 2 mm à 50 mm et une épaisseur de 0,2 mm à 10 mm.

**16.** Tube industriel selon l'une quelconque des revendications 13 à 15, comprenant en outre une couche comprenant au moins une résine sélectionnée dans le groupe constitué d'une résine fluorée, d'une résine de polyéthylène haute densité, d'une résine de polynaphtalate de butylène (PBN), d'une résine de polyamide aliphatique, d'une résine de polyamide aromatique, d'une résine de polyamide contenant un groupe métaxylène, d'un produit saponifié de copolymère d'éthylène-acétate de vinyle (EVOH) et d'une résine de polysulfure de phénylène (PPS).

**17.** Composition d'élastomère thermoplastique à base de polyamide [Z] comprenant 10 à 35 % en masse d'un polyamide [Z1] ayant un point de fusion (Tm) déterminé par calorimétrie à balayage différentiel (DSC) de 210 à 270 °C et une capacité calorifique de fusion ($\Delta$H) déterminée par DSC de 45 à 80 mJ/mg, et 65 à 90 % en masse d'une composition de résine à base de polyamide [Z2], à condition qu'une quantité totale de [Z1] et [Z2] soit de 100 % en masse, dans laquelle

la composition de résine à base de polyamide [Z2] est la composition d'élastomère thermoplastique à base de polyamide [Y2] selon la revendication 12.

**18.** Article moulé obtenu à partir de la composition d'élastomère thermoplastique à base de polyamide [Z] selon la revendication 17 par moulage par injection ou moulage par soufflage.

**19.** Soufflet de joint homocinétique pour automobile comprenant la composition d'élastomère thermoplastique à base de polyamide [Z] selon la revendication 17.

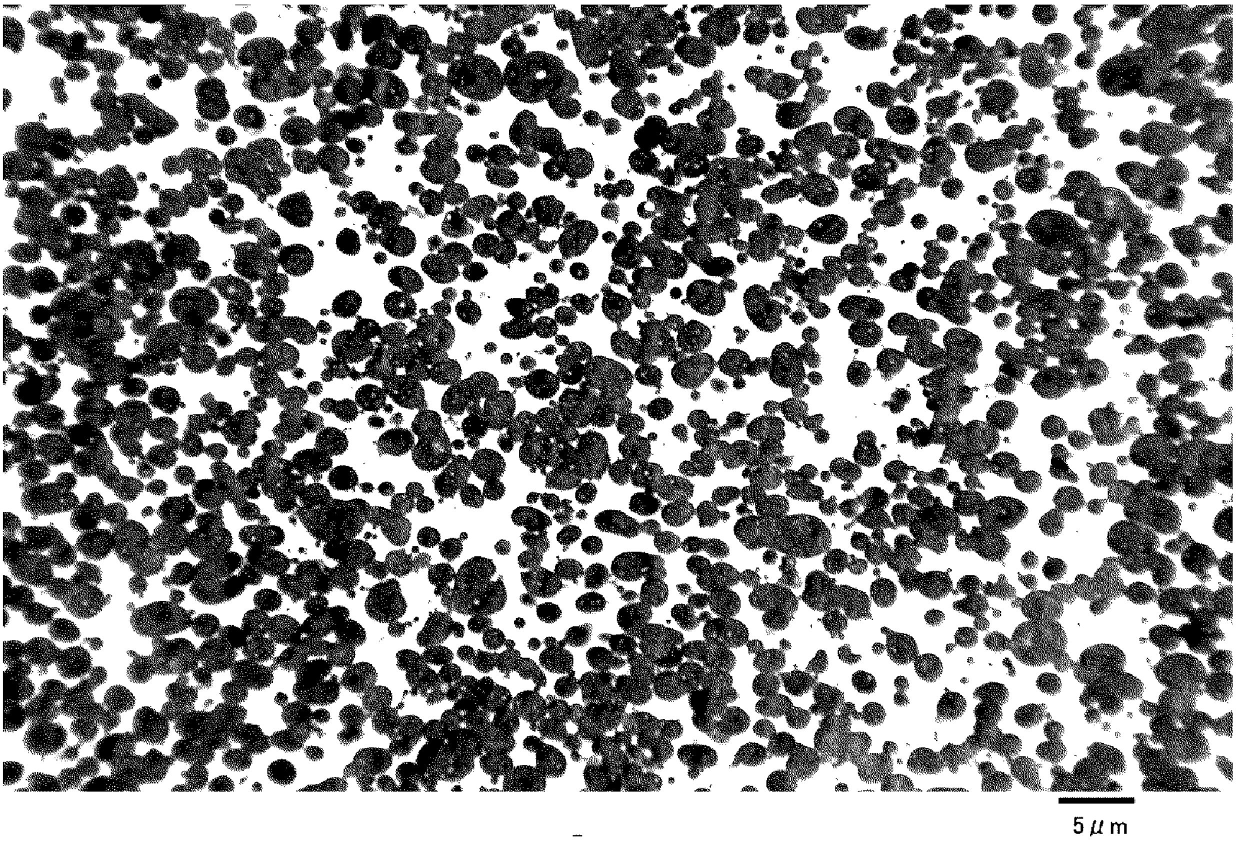


Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1698661 A1
- JP 63041554 A
- JP 8231770 A
- JP 2011148887 A
- WO 2006003973 A
- JP 2004217698 A
- JP 2001173672 A
- WO 2005102694 A
- JP 2012224085 A
- JP 2013095802 A
- JP 2010241897 A
- US 4311628 A
- US 2972600 A
- US 3287440 A